# FASCICULE DE BREVET EUROPEEN

(11) **EP 2 266 528 B1**
(45) Date de publication et mention de la délivrance du brevet: **11.09.2013**
(21) Numéro de dépôt: 10161415.4
(22) Date de dépôt: 29.04.2010
(51) Int. Cl.: A61K 8/58, A61K 8/31, A61K 8/34, A61K 8/37, A61Q 5/08, A61Q 5/10

(54) **Procédé de coloration et/ou de blanchiement des cheveux à base d'une composition comprenant une amino trialcoxysilane**
Verfahren zur Färbung und/oder Aufhellung für Keratinfasern Mittels amino trialkoxysilan
Process for colouring and/or bleaching human hair using a composition comprising an amino trialkoxysilane

(30) Priorité: 30.04.2009 FR 0952931; 30.04.2009 FR 0952914; 30.04.2009 FR 0952934
(43) Date de publication de la demande: 29.12.2010
(73) Titulaire: L'Oréal, 75008 Paris (FR)
(72) Inventeur: Charrier, Delphine, 75016, Paris (FR); Audousset, Marie-Pascale, 92600, Asnieres (FR); Hercouet, Leïla, 93360, Neuilly Plaisance (FR)
(74) Mandataire: Prevel, Estelle Nicole

(56) Documents cités:
- EP-A1- 1 298 135
- US-A1- 2006 110 351

## Description

La présente invention a pour objet un procédé d'éclaircissement et/ou de coloration de fibres kératiniques humaines, mettant en oeuvre en présence d'un agent oxydant, trois compositions, dont l'une, substantiellement anhydre ou aqueuse, comprend au moins un composé particulier amino-trialcoxy-silane.
Elle concerne enfin un dispositif à trois compartiments.

Parmi les méthodes de coloration des fibres kératiniques humaines, telles que les cheveux, on peut citer la coloration d'oxydation ou permanente. Plus particulièrement, ce mode de coloration met en oeuvre un ou plusieurs précurseurs de colorant d'oxydation, plus particulièrement une ou plusieurs bases d'oxydation éventuellement associées à un ou plusieurs coupleurs.

Habituellement, des bases d'oxydation sont choisies parmi les ortho- ou para-phénylènediamines, les ortho- ou para-aminophénols ainsi que des composés hétérocycliques. Ces bases d'oxydation sont des composés incolores ou faiblement colorés qui, associés à des produits oxydants, permettent d'accéder à des espèces colorées, par un processus de condensation oxydative.

Bien souvent, on fait varier les nuances obtenues avec ces bases d'oxydation en les associant à un ou plusieurs coupleurs, ces derniers étant choisis notamment parmi les méta-diamines aromatiques, les méta-aminophénols, les méta-diphénols et certains composés hétérocycliques, tels que des composés indoliques.

La variété des molécules mises en jeu au niveau des bases d'oxydation et des coupleurs permet l'obtention d'une riche palette de couleurs.

Le procédé de coloration consiste à mettre en contact le ou les précurseurs de colorants d'oxydation avec un agent oxydant, qui est de préférence le peroxyde d'hydrogène, dans des conditions alcalines. L'une des difficultés réside dans le fait que l'agent alcalin le plus couramment utilisé est l'ammoniaque, dont le rôle est d'ajuster le pH de la composition à un pH alcalin pour permettre la dégradation de l'agent oxydant. Ainsi l'oxygène formé provoque la condensation des précurseurs de colorants d'oxydation ainsi qu'un éclaircissement de la fibre dû à la dégradation de la mélanine présente. L'agent alcalinisant a également un autre rôle, celui de faire gonfler la fibre kératinique afin de favoriser la pénétration de l'oxydant ainsi que des colorants à l'intérieur de la fibre.

Or cet agent alcalinisant est très volatil, ce qui occasionne des désagréments à l'utilisateur du fait de l'odeur caractéristique forte, plutôt incommodante de l'ammoniac qui se dégage durant le procédé.

De plus, la quantité d'ammoniac dégagée nécessite l'emploi de teneurs plus importantes que nécessaires pour compenser cette perte. Cela n'est pas sans conséquence pour l'utilisateur qui reste non seulement incommodé par l'odeur mais qui peut également être confronté à des risques plus importants d'intolérance, comme par exemple une irritation du cuir chevelu (picotements).

Quant à l'option de purement et simplement remplacer en totalité ou en partie l'ammoniaque par un ou plusieurs autres agents alcalinisants classiques, celle-ci ne conduit pas à des compositions aussi efficaces que celles à base d'ammoniaque, notamment parce que ces agents alcalinisants ne conduisent pas un éclaircissement suffisant des fibres pigmentées en présence de l'agent oxydant.

Un autre mode de coloration employé est celui de la coloration directe ou semi-permanente. Ce procédé consiste à appliquer sur les fibres kératiniques des colorants directs qui sont des molécules colorées et colorantes, ayant une affinité pour les fibres, à laisser poser pour permettre aux molécules de pénétrer, par diffusion, à l'intérieur de la fibre, puis à les rincer.

Les colorants directs généralement employés sont choisis parmi les colorants directs nitrés benzéniques, anthraquinoniques, nitropyridiniques, azoïques, méthiniques, azométhiniques, xanthéniques, acridiniques, aziniques ou triarylméthaniques.

Ce mode de coloration ne nécessite pas l'emploi d'un agent oxydant à moins que l'on ne souhaite obtenir simultanément à la coloration, un éclaircissement de la fibre. Dans ce dernier cas, on procède comme pour la coloration d'oxydation, c'est-à-dire en mettant en contact les fibres kératiniques avec la composition tinctoriale en présence d'un agent oxydant, plus particulièrement le peroxyde d'hydrogène, en condition alcaline généralement en présence d'ammoniaque. On se retrouve donc également confronté aux mêmes difficultés que celles détaillées auparavant pour la coloration d'oxydation.

Outre les procédés de coloration, il est également courant de mettre en oeuvre des procédés d'éclaircissement dans lesquels on met en contact les fibres kératiniques avec une composition oxydante en conditions alcalines. Ces procédés consistent à uniquement dégrader la mélanine du cheveu, dans une plus ou moins forte mesure en fonction de l'agent oxydant choisi. Ainsi un sel peroxygéné conduit en général à un éclaircissement plus prononcé qu'en employant du peroxyde d'hydrogène seul en conditions alcalines. Cependant, quelque soit l'agent oxydant retenu, les procédés d'éclaircissement nécessitent l'emploi de peroxyde hydrogène en conditions alcalines et en particulier en présence d'ammoniaque pour former ou accélérer la formation d'oxygène. Par conséquent, on rencontre là encore, les mêmes difficultés que celles des procédés de coloration mis en oeuvre en présence d'un agent oxydant et d'ammoniaque.

L'un des objectifs de la présente invention est donc de proposer des compositions de coloration et/ou d'éclaircissement des fibres kératiniques humaines destinées à être mises en oeuvre en présence d'un agent oxydant qui ne présentent pas les inconvénients que les compositions existantes, dus à la présence de teneurs importantes en ammoniaque, tout en restant au moins aussi efficaces, tant sur le plan de l'éclaircissement que sur celui de la coloration, avec en particulier de bonnes performances en terme de chromaticité, de puissance et d'homogénéité

Ces buts et d'autres sont atteints par la présente invention qui a donc pour objet un procédé de coloration et/ou d'éclaircissement des fibres kératiniques humaines, dans lequel on met en contact lesdites fibres avec :
*une composition (A) substantiellement anhydre présentant une teneur en eau inférieure à 5% en poids ou aqueuse présentant une teneur en eau supérieure à 5% en poids, comprenant un ou plusieurs corps gras exempts de groupe acide carboxylique, un ou plusieurs tensioactifs,
* une composition (B) substantiellement anhydre présentant une teneur en eau inférieure à 5% en poids ou aqueuse présentant une teneur en eau supérieure à 5% en poids, comprenant un ou plusieurs composés aminosiliciés ne comprenant qu'un atome de silicium portant trois groupements alcoxy en C₁-C₄, de formule suivante (I) : 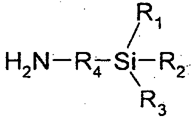 formule dans laquelle :
   - R₁, R₂, R₃ sont identiques
   - R₄ est un radical divalent de structure

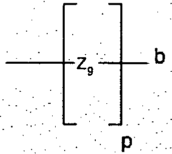

Dans laquelle :
■ Z₉ désigne un radical alkylène linéaire en C₁-C₂₀
■ p vaut 1
■ b représente la liaison à l'atome d'azote du groupement amino,
* une composition aqueuse (C) comprenant un ou plusieurs agents oxydants.

Le ou les composés organiques du silicium sont de préférence des organosilanes choisis parmi les composés de formule (IV) : 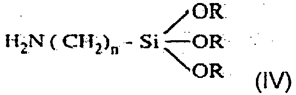 dans laquelle les radicaux R, identiques, sont choisis parmi les radicaux alkyle C₁-C₂, et n est un nombre entier de 1 à 6, de préférence de 2 à 4.

De préférence, les silanes ou les siloxanes sont solubles dans l'eau et encore plus préférentiellement solubles à la concentration de 2%, mieux à la concentration de 5% et encore mieux à la concentration de 10% en poids dans l'eau à la température de 25°C±5°C et à la pression atmosphérique.Par soluble, on entend la formation d'une phase macroscopique unique.

Le procédé de la présente invention permet d'obtenir une coloration des cheveux avec des propriétés tinctoriales très satisfaisantes, notamment en terme d'intensité, de puissance, d'homogénéité, de chromaticité, de sélectivité et une bonne résistance de cette couleur aux agents extérieurs tels que la résistance aux shampoings, à la sueur, aux intempéries.

On a également remarqué que le procédé selon l'invention confère aux cheveux ainsi traités de bonnes propriétés coiffantes telles que du volume et du corps, un toucher doux et lisse, et cela même lorsqu'il est appliqué sur des cheveux préalablement sensibilisés par un traitement chimique.

Un autre objet de l'invention est constitué par un dispositif à plusieurs compartiments comprenant dans un premier compartiment une composition (A), dans un second compartiment une composition (B) et dans un troisième compartiment une composition (C) telles que décrites précédemment.

La présente invention a aussi pour objet une composition prête à l'emploi comprenant un ou plusieurs composés organiques du silicium tels que définis précédemment et un ou plusieurs colorants, de préférence un ou plusieurs précurseurs de colorant, un ou plusieurs agents oxydants et un ou plusieurs polymères cationiques et / ou un ou plusieurs tensioactifs non ioniques oxyalkylénés ou glycérolés.

D'autres caractéristiques et avantages de l'invention apparaîtront plus clairement à la lecture de la description et des exemples qui suivent.

Dans ce qui va suivre, et à moins d'une autre indication, les bornes d'un domaine de valeurs sont comprises dans ce domaine.

Les fibres kératiniques humaines traitées par le procédé selon l'invention sont de préférence les cheveux.

Par ailleurs, comme indiqué auparavant, la(les) composition(s) (A) et / (B) est(sont) une(des) composition(s) substantiellement anhydre(s) ou aqueuse(s).

Dans le sens de la présente invention, une composition aqueuse comprend plus de 5 % en poids d'eau, de préférence plus de 10 % en poids d'eau, et de manière encore plus avantageuse plus de 20 % en poids d'eau.

Selon un mode de réalisation particulier de l'invention, lorsque la(les) composition(s) (A) et / ou (B) est(sont) aqueuse(s), la teneur en eau varie de préférence de 5 à 95 % en poids encore plus préférentiellement de 25 à 90 % en poids, mieux de 40 à 85 % en poids, par rapport au poids de ladite composition.

Selon un autre mode de réalisation particulier de l'invention, lorsque la(les) composition(s) (A) et / ou (B) est(sont) aqueuse(s), la teneur en eau varie de préférence de 30 à 78 % en poids encore plus préférentiellement de 40 à 70 % en poids, mieux de 45 à 60 % en poids, par rapport au poids de ladite composition.

La(les) composition(s) (A) et / ou (B) peu(ven)t alors également comprendre un ou plusieurs solvants organiques.

A titre de solvant organique, on peut par exemple citer, les alcanols, linéaires ou ramifiés, en C₂-C₄, tels que l'éthanol et l'isopropanol ; le glycérol ; les glycols et éthers de glycols comme le 2-butoxyéthanol, le propylèneglycol, le dipropylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Le ou les solvants peuvent être présents dans des proportions allant habituellement de 1 à 40 % en poids par rapport au poids de ladite composition, et de préférence de 5 à 30 % en poids.

Au sens de l'invention, une composition substantiellement anhydre présente une teneur en eau inférieure à 5 % en poids, plus particulièrement inférieure à 2 %, de préférence inférieure à 1% en poids par rapport au poids de ladite composition. Il est à noter qu'il s'agit plus particulièrement d'eau liée, comme l'eau de cristallisation des sels ou des traces d'eau absorbée par les matières premières utilisées dans la réalisation des compositions selon l'invention.

Il est également précisé, que compte tenu de l'usage qui en est fait, les compositions mises en oeuvre dans le procédé selon l'invention ne comprennent pas d'ingrédients qui la ou les rendraient inutilisables pour la coloration et/ou l'éclaircissement des fibres kératiniques humaines. Ainsi, les ingrédients qu'elles comprennent sont cosmétiquement acceptables.

Comme indiqué précédemment, la composition (A) substantiellement anhydre ou aqueuse comprend un ou plusieurs corps gras exempts de groupe acide carboxylique, un ou plusieurs tensioactifs.

Par corps gras, on entend, un composé organique insoluble dans l'eau à température ordinaire (25°C) et à pression atmosphérique (760 mm de Hg) (solubilité inférieure à 5% et de préférence à 1% encore plus préférentiellement à 0.1%). En outre, les corps gras sont solubles dans les solvants organiques dans les mêmes conditions de température et de pression, comme par exemple le chloroforme, l'éthanol ou le benzène.

Selon l'invention, les corps gras sont choisis parmi les composés liquides ou pâteux à température ambiante et à pression atmosphérique.

Plus particulièrement, les corps gras sont choisis parmi les alcanes, les alcools gras, les esters d'acide gras, les esters d'alcool gras, les huiles minérales, végétales, animales ou synthétiques, les silicones, les cires.

Il est rappelé qu'au sens de l'invention, les alcools et esters gras présentent plus particulièrement au moins un groupement hydrocarboné, linéaire ou ramifié, saturé ou insaturé, comprenant 6 à 30 atomes de carbone, éventuellement substitué, en particulier par un ou plusieurs groupements hydroxyle (en particulier 1 à 4). S'ils sont insaturés, ces composés peuvent comprendre une à trois double-liaisons carbone-carbone, conjuguées ou non.

En ce qui concerne les alcanes, ces derniers comprennent de 6 à 30 atomes de carbone, sont linéaires. A titre d'exemple, on peut citer l'hexane, le dodécane.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol^{®} 810, 812 et 818 par la société Dynamit Nobel, l'huile de jojoba, l'huile de beurre de karité ;
- les hydrocarbures linéaires ou ramifiés, d'origine minérale ou synthétique, tels que les huiles de paraffine, volatiles ou non, et leurs dérivés, la vaseline, l'huile de vaseline, les polydécènes, le polyisobutène hydrogéné tel que Parléam^{®} ; les isoparaffines comme l'isohexadécane et l'isodécane.
- les alcools gras sont saturés ou insaturés, linéaires ou ramifiés, et comportent de 8 à 30 atomes de carbone, on peut citer l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ; comme huiles fluorées, on peut citer aussi le perfluorométhylcyclopentane et le perfluoro-1,3 diméthylcyclohexane, vendus sous les dénominations de "FLUTEC^{®} PC1" et "FLUTEC^{®} PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050^{®}" et "PF 5060^{®}" par la Société 3M, ou encore le bromoperfluorooctyle vendu sous la dénomination "FORALKYL^{®}" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052^{®}" par la Société 3M.

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les esters sont les esters de mono ou polyacides aliphatiques saturés ou insaturés, linéaires ou ramifiés en C₁-C₂₆ et de mono ou polyalcools aliphatiques saturés ou insaturés, linéaires ou ramifiés en C₁-C₂₆, le nombre total de carbone des esters étant supérieur ou égal à 10.

Parmi les monoesters , on peut citer le béhénate de dihydroabiétyle ; le béhénate d'octyldodécyle ; le béhénate d'isocétyle ; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, de mirystyle, de stéaryle le stéarate d'hexyle, le stéarate de butyle, le stéarate d'isobutyle ; le malate de dioctyle, le laurate d'hexyle, le laurate de 2-hexyldécyle.

Toujours dans le cadre de cette variante, on peut également utiliser les esters d'acides di ou tricarboxyliques en C₄-C₂₂ et d'alcools en C₁-C₂₂ et les esters d'acides mono di ou tricarboxyliques et d'alcools di, tri, tétra ou pentahydroxy en C₂-C₂₆.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

La composition peut également comprendre, à titre d'ester gras, des esters et di-esters de sucres d'acides gras en C₆-C₃₀, de préférence en C₁₂-C₂₂. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en C₆-C₃₀, de préférence en C₁₂-C₂₂, linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléo-palmitate, oléo-stéarate, palmito-stéarate.

Plus particulièrement, on utilise les mono- et di- esters et notamment les mono- ou di- oléate, stéarate, béhénate, oléopalmitate, linoléate, linolénate, oléostéarate, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di- et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

Les silicones utilisables dans les compositions cosmétiques de la présente invention, sont des silicones volatiles ou non volatiles, cycliques, linéaires ou ramifiées, modifiées ou non par des groupements organiques, ayant une viscosité de 5.10⁻⁶ à 2,5m²/s à 25°C et de préférence 1.10⁻⁵ à 1m²/s.

Les silicones utilisables conformément à l'invention peuvent se présenter sous forme d'huiles, de cires, de résines ou de gommes.

De préférence, la silicone est choisie parmi les polydialkylsiloxanes, notamment les polydiméthylsiloxanes (PDMS), et les polysiloxanes organo-modifiés comportant au moins un groupement fonctionnel choisi parmi les groupements poly(oxyalkylène), les groupements aminés et les groupements alcoxy.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:
(i) les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE^{®} 7207 par UNION CARBIDE ou SILBIONE^{®} 70045 V2 par RHODIA, le décaméthylcyclopentasiloxane commercialisé sous le nom de VOLATILE SILICONE^{®} 7158 par UNION CARBIDE, et SILBIONE® 70045 V5 par RHODIA, ainsi que leurs mélanges.

On peut également citer les cyclocopolymères du type diméthylsiloxanes/méthylalkylsiloxane, tel que la SILICONE VOLATILE^{®} FZ 3109 commercialisée par la société UNION CARBIDE, de formule : avec 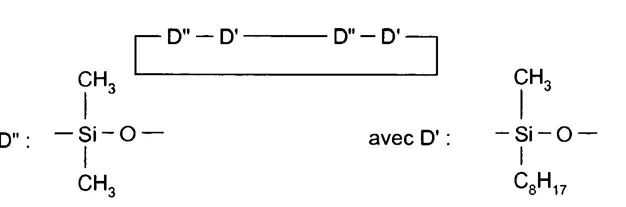 On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les polydialkylsiloxanes volatiles linéaires ayant 2 à 9 atomes de silicium et présentant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 mm²/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl (C₁-C₂₀) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :
R₂SiO_{2/2}, R₃SiO_{1/2}, RSiO_{3/2} et SiO_{4/2}
dans lesquelles R représente un alkyl possédant 1 à 16 atomes de carbone. Parmi ces produits, ceux particulièrement préférés sont ceux dans lesquels R désigne un groupe alkyle inférieur en C₁-C₄, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre, les silicones décrites ci-dessus les silicones organomodifiées peuvent être des polydiaryl siloxanes, notamment des polydiphénylsiloxanes, et des polyalkylarylsiloxanes fonctionnalisés par les groupes organofonctionnels mentionnés précédemment.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl (C₁₂)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.

De préférence, le corps gras est un composé liquide à la température de 25°C et à la pression atmosphérique.

De préférence, le corps gras est choisi parmi l'huile de vaseline, l'huile de paraffine, les polydécènes, les esters d'acides gras, de préférence liquides, ou leurs mélanges, et encore plus préférentiellement l'huile de vaseline, de paraffine et les esters d'acides gras, et leurs mélanges

La composition (A) présente une teneur en corps gras comprise avantageusement entre 10 et 99 % en poids, par rapport au poids de la composition, de préférence entre 20 et 90 % en poids, préférentiellement entre 25 et 80% mieux entre 30 et 70% en poids.

La composition (A) comprend également un ou plusieurs tensioactifs.

De préférence, le ou les tensioactifs sont choisis parmi les tensioactifs non ioniques ou parmi les tensioactifs anioniques.

Les tensioactifs anioniques sont plus spécialement choisis parmi les sels (en particulier sels de métaux alcalins, notamment de sodium, sels d'ammonium, sels d'amines, sels d'aminoalcools ou sels de métaux alcalino-terreux comme le magnésium) des composés suivants :
- les alkylsulfates, les alkyléthersulfates, alkylamidoéthersulfates, alkylaryl-polyéthersulfates, monoglycérides sulfates ;
- les alkylsulfonates, alkylamidesulfonates, alkylarylsulfonates, α-oléfine-sulfonates, paraffine-sulfonates ;
- les alkylphosphates, les alkylétherphosphates;
- les alkylsulfosuccinates, les alkyléthersulfosuccinates, les alkylamide-sulfosuccinates; les alkylsulfosuccinamates ;
- les alkylsulfoacétates ;
- les acylsarcosinates ; les acyliséthionates et les N-acyltaurates ;
- les sels d'acides gras tels que les acides oléique, ricinoléique, palmitique, stéarique, les acides d'huile de coprah ou d'huile de coprah hydrogénée ;
- les sels d'acides d'alkyl D galactoside uroniques ;
- les acyl-lactylates ;
- les sels des acides alkyléther carboxyliques polyoxyalkylénés, des acides alkylaryléther carboxyliques polyoxyalkylénés, des acides alkylamidoéther carboxyliques polyoxyalkylénés, en particulier ceux comportant de 2 à 50 groupements oxyde d'éthylène ;
- et leurs mélanges.

Notons que le radical alkyle ou acyle de ces différents composés comporte avantageusement de 6 à 24 atomes de carbone, et de préférence de 8 à 24 atomes de carbone, et le radical aryle désignant de préférence un groupement phényle ou benzyle.

Les tensioactifs non ioniques sont plus particulièrement choisis parmi les tensioactifs non ioniques mono ou poly- oxyalkylénés, mono- ou poly- glycérolés.

Par tensioactif oxyalkylénés ou glycérolé, on entend au sens de la présente invention un composé comportant une ou plusieurs chaînes hydrocarbonée comportant au moins 6 atomes de carbone et au moins un groupement de structure

-CH₂-(C(H)t(CH₂R₁)ₙ)_{q}-CH₂ₚ-O-

Avec n ou pou q désignant indépendamment l'un de l'autre 0 ou 1 t désigne 1 ou 2
Et R₁ désignant un atome d'hydrogène ou un radical hydroxy.
Ces groupements peuvent être dits oxyéthyléné (q=0, p=1), oxypropyléné(q=1, n=0 t=2 p=1 ou q=1 t= 1 n=1 R1=H) ou glycérolé((q=1, n=0 t=2 p=1 ou q=1 t= 1 n=1 R1=OH)

Les motifs oxyalkylénés sont plus particulièrement des motifs oxyéthylénés, oxypropylénés, ou leur combinaison, de préférence oxyéthylénés.
A titre d'exemples de tensioactifs non ioniques oxyalkylénés, on peut citer :
- les alkyl(C₈-C₂₄)phénols oxyalkylénés, de préférence les alkyl(C₈-C₁₈)phénols oxyalkylénés,
- les alcools gras, de préférence en C₈-C₃₀, saturés ou non, linéaires ou ramifiés, oxyalkylénés,
- les amides gras, de préférence en C₈-C₃₀, saturés ou non, linéaires ou ramifiés, oxyalkylénés,
- les esters d'acides gras, de préférence en C₈-C₃₀, saturés ou non, linéaires ou ramifiés, du sucrose oxyalkylénés,
- les esters d'acides gras, de préférence en C₈-C₃₀, saturés ou non, linéaires ou ramifiés, et de polyéthylèneglycols,
- les esters d'acides en C₆-C₃₀, de préférence en C₈-C₃₀, saturés ou non, linéaires ou ramifiés, et de sorbitane polyoxyéthylénés,
- les huiles végétales oxyéthylénées, saturées ou non,
- les condensats d'oxyde d'éthylène et/ou d'oxyde de propylène, entre autres, seuls ou en mélanges.
A titre d'exemples de tensioactifs non ioniques glycérolés, on peut citer :
- les alcools gras, de préférence en C₈-C₃₀, saturés ou non, linéaires ou ramifiés, glycérolés,
- les amides gras, de préférence en C₈-C₃₀, saturés ou non, linéaires ou ramifiés, glycérolés
   et leurs mélanges.

Selon un mode de réalisation particulier de l'invention, le nombre moyen de motifs oxyalkylénés est avantageusement compris entre 2 et 150 motifs. De préférence, il s'agit de motifs oxyéthylénés, oxypropylénés ou leurs mélanges.

En ce qui concerne les tensioactifs glycérolés, ils comportent de préférence en moyenne 1 à 20 groupements glycérol et en particulier 1,5 à 5.

Selon un autre mode de réalisation particulier de l'invention, les tensioactifs présentent un nombre de moles d'oxyde d'éthylène et/ou de propylène compris entre 1 et 90, plus particulièrement entre 1 et 50, et de préférence entre 2 et 30. De manière avantageuse, les tensioactifs non ioniques ne comprennent pas de motifs oxypropylénés.

Conformément à un mode de réalisation préféré de l'invention, les tensioactifs non ioniques oxyalkylénés sont choisis parmi les alcools en C₈-C₃₀, oxyéthylénés, les esters d'acides en C₈-C₃₀, saturés ou non, linéaires ou ramifiés, et de sorbitane polyoxyéthylénés.

A titre de tensioactifs non ioniques mono- ou poly- glycérolés, on utilise de préférence les alcools en C₈-C₄₀, mono- ou poly- glycérolés.

En particulier, les alcools en C₈-C₄₀ mono- ou poly- glycérolés correspondent à la formule suivante :

RO-[CH₂-CH(CH₂OH)-O]ₘ-H

dans laquelle R représente un radical alkyle ou alcényle, linéaire ou ramifié, en C₈-C₄₀, de préférence en C₈-C₃₀, et m représente un nombre allant de 1 à 30 et de préférence de 1 à 10.

A titre d'exemple de composés convenables dans le cadre de l'invention, on peut citer, l'alcool laurique à 4 moles de glycérol (nom INCI: POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool peut représenter un mélange d'alcools au même titre que la valeur de m représente une valeur statistique, ce qui signifie que dans un produit commercial peuvent coexister plusieurs espèces d'alcools gras polyglycérolés sous forme d'un mélange.

Parmi les alcools mono- ou poly-glycérolés, on préfère plus particulièrement utiliser l'alcool en C₈/C₁₀ à une mole de glycérol, l'alcool en C₁₀/C₁₂ à 1 mole de glycérol et l'alcool en C₁₂ à 1,5 mole de glycérol.

De préférence, le tensioactif présent dans la composition (A) est un tensioactif non ionique ou un mélange de tensioactifs non ioniques.

La teneur en tensioactifs dans la composition (A) représente plus particulièrement de 0,1 à 50 % en poids, de préférence de 0,5 à 30 % en poids par rapport au poids de la composition (A).

La composition substantiellement anhydre ou aqueuse (A) peut éventuellement comprendre un ou plusieurs agents alcalins.

L'agent alcalin est plus particulièrement choisi parmi les amines organiques ou leurs sels, les bases minérales, les sels d'ammonium, ou leurs mélanges.

Avantageusement, les amines organiques employées comme agent alcalin sont choisies parmi les amines organiques dont le pK_{b} à 25°C est inférieur à 12, de préférence inférieur à 10, et encore plus préférentiellement inférieur à 6.

Il est à noter qu'il s'agit du pK_{b} correspondant à la fonction de basicité la plus élevée.

De préférence, la ou les amines organiques selon l'invention ne comportent pas de chaîne grasse comprenant plus de 10 atomes de carbone.

En particulier, l'amine organique comprend une ou deux fonctions amine primaire, secondaire ou tertiaire, et un ou plusieurs groupements alkyle, linéaires ou ramifiés, en C₁-C₈ porteurs d'un ou plusieurs radicaux hydroxyle.

Conviennent en particulier à la réalisation de l'invention les amines organiques choisies parmi les alcanolamines telles que les mono-, di- ou tri- alcanolamines, comprenant un à trois radicaux hydroxyalkyle, identiques ou non, en C₁-C₄.

Parmi des composés de ce type, on peut citer la monoéthanolamine, la diéthanolamine, la triéthanolamine, la monoisopropanolamine, la diisopropanolamine, la N-diméthylaminoéthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol, le tris-hydroxyméthylaminométhane.

Conviennent également les amines organiques de formule suivante : 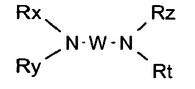 dans laquelle W est un reste alkylène en C₁-C₆ éventuellement substitué par un groupement hydroxyle ou un radical alkyle en C₁-C₆ ; Rx, Ry, Rz et Rt, identiques ou différents, représentent un atome d'hydrogène, un radical alkyle en C₁-C₆ ou hydroxyalkyle en C₁-C₆, aminoalkyle en C₁-C₆.

On peut citer à titre d'exemple de telles amines, le 1,3 diaminopropane, le 1,3 diamino 2 propanol, la spermine, la spermidine.

L'amine organique peut également être choisie parmi les acides aminés.

Plus particulièrement, les acides aminés utilisables sont d'origine naturelle ou de synthèse, sous leur forme L, D, ou racémique et comportent au moins une fonction acide choisie plus particulièrement parmi les fonctions acides carboxyliques, sulfoniques, phosphoniques ou phosphoriques. Les acides aminés peuvent se trouver sous forme neutre ou ionique.

A titre d'acides aminés utilisables dans la présente invention, on peut notamment citer l'acide aspartique, l'acide glutamique, l'alanine, l'arginine, l'ornithine, la citrulline, l'asparagine, la carnitine, la cystéine, la glutamine, la glycine, l'histidine, la lysine, l'isoleucine, la leucine, la méthionine, la N-phénylalanine, la proline, la serine, la taurine la thréonine, le tryptophane, la tyrosine et la valine.

De manière avantageuse, les acides aminés sont des acides aminés basiques comprenant une fonction amine supplémentaire éventuellement incluse dans un cycle ou dans une fonction uréido.

De tels acides aminés basiques sont choisis de préférence parmi ceux répondant à la formule (II) suivante : 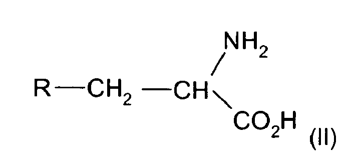 où R désigne un groupe choisi parmi :

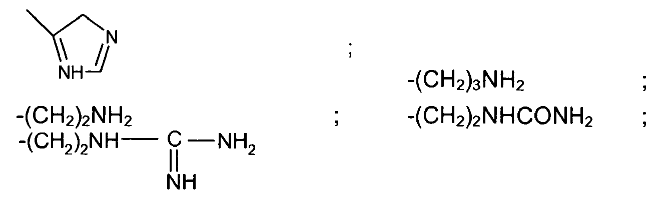

Les composés correspondants à la formule (II) sont l'histidine, la lysine, l'arginine, l'ornithine, la citrulline.

Selon une variante préférée de l'invention, l'acide aminé est basique, et plus particulièrement choisi parmi l'arginine, la lysine, l'histidine, ou leurs mélanges.

L'amine organique peut aussi être choisie parmi les amines organiques de type hétérocycliques On peut en particulier citer, outre l'histidine déjà mentionnée dans les acides aminés, la pyridine, la pipéridine, l'imidazole, le triazole, le tétrazole, le benzimidazole.

L'amine organique peut aussi être choisie parmi les dipeptides d'acides aminés. A titre de dipeptides d'acides aminés utilisables dans la présente invention, on peut notamment citer la carnosine, l'anserine et la baleine.

L'amine organique peut encore être choisie parmi les composés comportant une fonction guanidine. A titre d'amines d'amines de ce type utilisables dans la présente invention, on peut notamment citer outre l'arginine déjà mentionnée à titre d'acide aminé, la créatine, la créatinine, la 1,1-diméthylguanidine, 1,1-diéthylguanidine, la glycocyamine, la metformine, l'agmatine, la n-amidinoalanine, l'acide 3-guanidinopropionique, l'acide 4-guanidinobutyrique et l'acide 2-([amino(imino)methyl] amino)ethane-1-sulfonique.

On peut également mettre en oeuvre des sels des amines précitées comme les sels organiques ou inorganiques d'une amine organique telle que décrite ci-dessous.

De préférence, les sels organiques sont choisis parmi les sels d'acides organiques tels que les citrates, les lactates, les glycolates, les gluconates, les acétates, les propionates, les fumarates, les oxalates et les tartrates.

De préférence, les sels inorganiques sont choisis parmi les halogénohydrates (chlorhydrates par exemple), les carbonates, les hydrogénocarbonates, les sulfates, les hydrogénophosphates et les phosphates.

La ou les bases inorganiques sont choisies parmi celles possédant dans sa structure un ou plusieurs éléments des colonnes 1 à 13 du tableau périodique des éléments autre que l'hydrogène, ne comportant pas simultanément d'atome(s) de carbone et d'hydrogène. Selon un mode de réalisation particulier de l'invention, la base inorganique contient un ou plusieurs éléments des colonnes 1 et 2 du tableau périodique des éléments autre que l'hydrogène.

Dans une variante préférée la base inorganique présente la structure suivante :

(Z₁^{x-})ₘ(Z₂^{y+})ₙ

Dans laquelle
Z₂ désigne un métal des colonnes 1 à 13 du tableau périodique des éléments, de préférence 1 ou 2, comme le sodium ou le potassium ;
Z₁^{x-} désigne un anion choisi parmi les ions CO₃²⁻, OH⁻, HCO₃²⁻, SiO₃²⁻, HPO₄²⁻, PO₄³⁻, B₄O₇²⁻, de préférence parmi les ions CO₃²⁻, OH⁻, SiO₃²⁻ ;
x désigne 1 , 2 ou 3 ;
y désigne 1, 2, 3 ou 4 ;
m et n désignent indépendamment l'un de l'autre 1, 2, 3 ou 4 ;
avec n.y=m.x.

De préférence, la base inorganique correspond à la formule suivante (Z₁^{x-})ₘ(Z₂^{y+})ₙ, dans laquelle Z₂ désigne un métal des colonnes 1 et 2, du tableau périodique des éléments ; Z₁^{x-} désigne un anion choisi parmi les ions CO₃²⁻, OH⁻, SiO₃²⁻, x vaut 1, y désigne 1 ou 2, m et n désignent indépendamment l'un de l'autre 1 ou 2 avec n.y=m.x.

A titre de base inorganique utilisable selon l'invention on peut citer, le carbonate de sodium, le carbonate de potassium, la soude, la potasse, le métasilicate de sodium, le métasilicate de potassium. De préférence la base inorganique est un carbonate alcalin.

Les sels d'ammonium sont de préférence choisis parmi les sels d'acide suivants : acétate, carbonate, bicarbonate, chlorure, citrate, nitrate, nitrite, phosphate, sulfate.

De manière particulièrement préférée on utilisera le carbonate d'ammonium.

De préférence, l'agent alcalin est choisi parmi les alcanolamines, les acides aminés basiques et les hydroxydes ou carbonates de métaux alcalins. Plus particulièrement, l'agent alcalin est choisi parmi les alcanolamines éventuellement en mélange avec les acides aminés basiques, les hydroxydes ou les carbonates de métaux alcalins.

Selon un mode de réalisation particulièrement avantageux de l'invention, l'agent alcalin est la monoéthanolamine utilisée seule ou en mélange avec les agents alcalins précités ; en particulier avec une base minérale telle que par exemple l'hydroxyde de sodium ou le carbonate de potassium, et/ou encore avec un acide aminé basique comme en particulier l'arginine.

De manière avantageuse, si la composition (A) comprend un ou plusieurs agents alcalins, il(s) est(sont) présents à une teneur variant de 0,01 à 30 % en poids, de préférence de 0,1 à 20 % en poids et de manière encore plus préférée, de 0,1 à 10% en poids par rapport au poids de ladite composition.

La composition substantiellement anhydre ou aqueuse (A) peut également renfermer divers adjuvants utilisés classiquement dans le domaine, tels que des polymères anioniques, cationiques, non ioniques, amphotères, zwitterioniques ou leurs mélanges ; des agents épaississants minéraux, et en particulier des charges telles que des argiles, le talc ; des agents épaississants organiques, avec en particulier les épaississants associatifs polymères anioniques, cationiques, non ioniques et amphotères ; des agents épaississants minéraux choisis notamment parmi les argiles organophiles, les silices pyrogénées ; des agents antioxydants ; des agents de pénétration ; des agents séquestrants ; des parfums ; des agents dispersants ; des agents filmogènes ; des céramides ; des agents conservateurs ; des agents opacifiants ; des agents conditionneurs avec en particulier des polymères cationiques.

Les adjuvants ci dessus sont en général présents en quantité comprise pour chacun d'eux entre 0,01 et 20 % en poids par rapport au poids de la composition (A).

De préférence, la composition (A) ne comprend pas de persels.

Le procédé selon l'invention est également mis en oeuvre en présence d'une composition substantiellement anhydre ou aqueuse (B) comprenant un ou plusieurs composés de formule (I) précitée.

A titre d'exemples de composés de formule (I) convenant à la mise en oeuvre de l'invention, on peut citer les composés suivants

| | |
|---|---|
| | 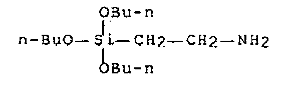 |
| | 894393-40-9 Ethanamine, 2-(tributoxysilyl)- |
| 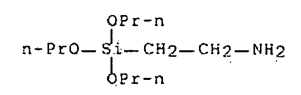 | 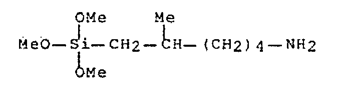 |
| 894393-33-0 Ethanamine, 2-(tripropoxysilyl)- | 852566-95-1 1-Hexanamine, 5-methyl-6-(trimethoxvsilyl)- |
| 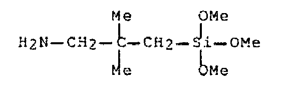 | |
| 848941-45-7 1-Propanamine, 2,2-dimethyl-3- (trimethoxysilyl)- | |
| | 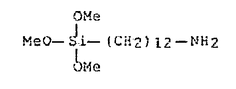 |
| | 771581-51-2 1-Dodecanamine, 12-(trimethoxysilyl)- |
| | 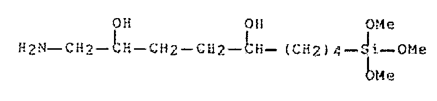 |
| | 587877-12-1 2,5-Nonanediol, 1-amino-9-(trimethoxysilyl)- |
| 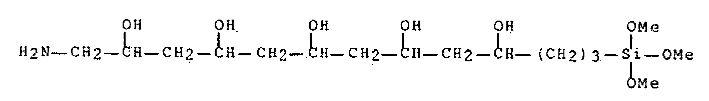 | |
| 587877-24-5 2,4,6,8,10-Tridecanepentol, 1-amino-13-(trimethoxysilyl) | |
| 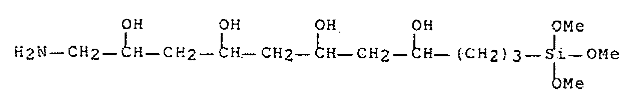 | |
| 587877-22-3 2,4,6,8-Undecanetetrol, 1-amino-11-(trimethoxysilyl) | |
| 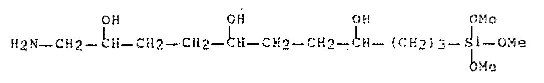 | |
| 587877-14-3 2,5,8-Undecanetriol, 1-amino-11-(trimethoxysilyl)- | |
| 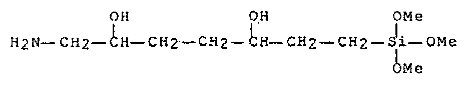 | |
| 587877-10-9 2,5-Heptanediol, 1-amino-7-(trimethoxysilyl)- | |
| 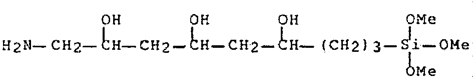 | 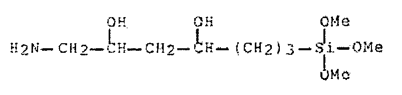 |
| 587877-08-5 2,4,6-Nonanetriol, 1-amino-9- (trimethoxysilyl)- | 587877-06-3 2,4-Heptanediol, 1-amino-7-(trimethoxysilyl)- |
| 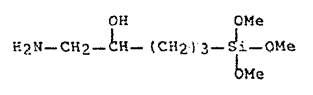 | 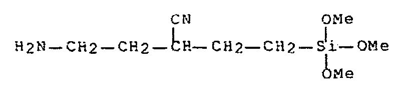 |
| 587877-04-1 2-Pentanol, 1-amino-5-(trimethoxysilyl)- | 587876-76-4 Butanenitrile, 2-(2-aminoethyl)-4-(trimethoxysilyl) |
| | |
| | 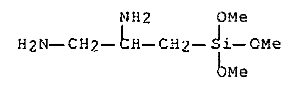 |
| | 402790-28-7 1,2-Propanediamine, 3-(trimethoxysilyl)- |
| | 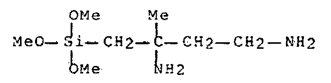 |
| | 331443-68-6 1,3-Butanediamine, 3-methyl-4-(trimethoxysilyl)- |
| | 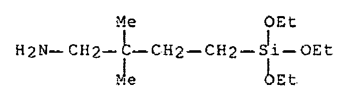 |
| | 327024-67-9 4-Amino-3,3-dimethylbutyltriethoxysilane |
| 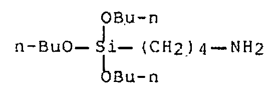 | |
| 314733-26-1 1-Butanamine, 4-(tributoxysilyl)- | |
| 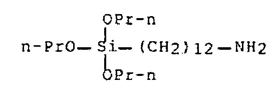 | 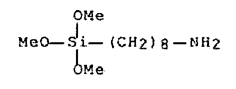 |
| 253596-69-9 1-Dodecanamine, 12-(tripropoxysilyl) | 253596-68-8 1-Octanamine, 8-(trimethoxysilyl) |
| | 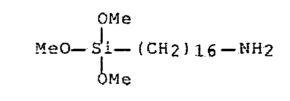 |
| | 193157-95-8 16-Aminohexadecyltrimethoxysilane |
| 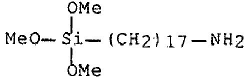 | 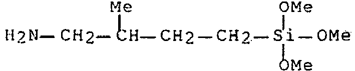 |
| 163193-89-3 | 157923-78-9 |
| (17-Aminoheptadecyl)trimethoxysilane | 4-Amino-3-methylbutyltrimethoxysilane |
| 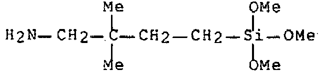 | |
| 157923-74-5 | |
| 4-(Trimethoxysilyl)-2,2-dimethylbutanamine | |
| | 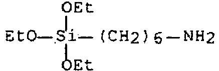 |
| | 134821-45-7 |
| | 1-Hexanamine, 6-(triethoxysilyl)- |
| 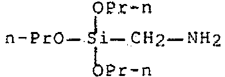 | |
| 131535-65-4 Methanamine, 1-(tripropoxysilyl)- | |
| | 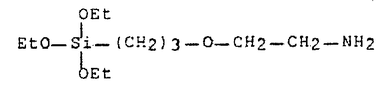 |
| | 126552-43-0 2-[3-(Triethoxysilyl)propoxy]ethylamine |
| | 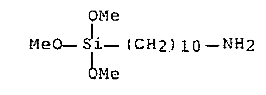 |
| | 120183-15-5 (10-Aminodecyl)trimethoxysilane |
| 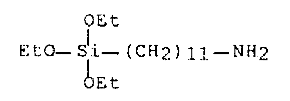 | |
| 116821-45-5 11-(Aminoundecyl)triethoxysilane | |
| | 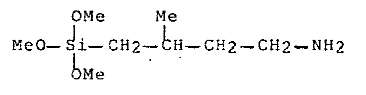 |
| | 106890-59-9 1-Butanamine, 3-methyl-4-(trimethoxysilyl) |
| 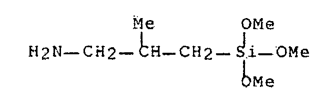 | |
| 99503-87-4 1-Propanamine, 2-methyl-3-(trimethoxysilyl)- | |
| | |
| 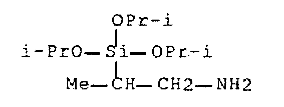 | 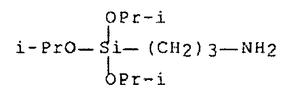 |
| 94989-07-8 1-Propanamine, 2-[tris(1-methylethoxy)silyl]- | 94989-06-7 3-Aminopropyltriisopropoxysilane |
| | 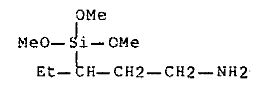 |
| | 92116-16-0 3-(Trimethoxysilyl)-1-pentanamine |
| 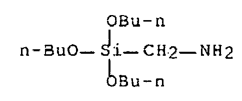 | |
| 84869-17-0 Aminomethyltributoxysilane | |
| 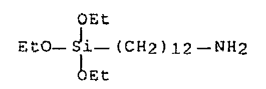 | 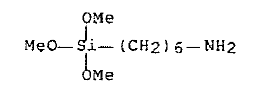 |
| 83943-65-1 1-Dodecanamine, 12-(triethoxysilyl) | 83943-64-0 1-Hexanamine, 6-(trimethoxysilyl) |
| | 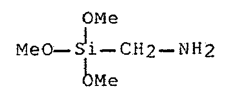 |
| | 71408-48-5 Methanamine, 1-(trimethoxysilyl)- |
| 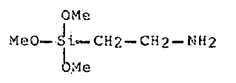 | |
| 65644-31-7 2-(Trimethoxysilyl)ethylamine | |
| | 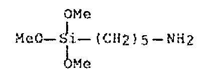 |
| | 54894-82-5 1-Pentanamine, 5-(trimethoxysilyl)- |
| | 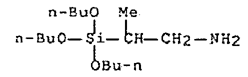 |
| | 53813-14-2 1-Propanamine, 2-(tributoxysilyl)- |
| | 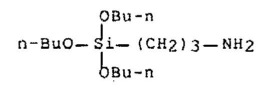 |
| | 52340-01-9 1-Propanamine, 3-(tributoxysilyl)- |
| 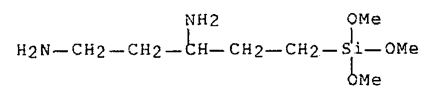 | 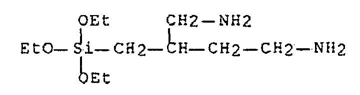 |
| 51279-08-4 1,3-Pentanediamine, 5-(trimethoxysilyl)- | 51279-07-3 1,4-Butanediamine, 2-[(triethoxysilyl)methyl]- |
| 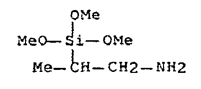 | |
| 50602-95-4 1-Propanamine, 2-(trimethoxysilyl)- | )ethyl]triethoxysilane |
| | 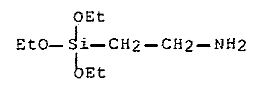 |
| | 45074-31-5 2-Aminoethyltriethoxysilane |
| 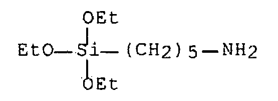 | |
| 1067-48-7 1-Pentanamine, 5-(triethoxysilyl)- | |
| 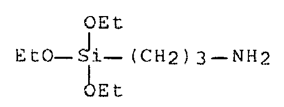 | |
| 919-30-2 3-Aminopropyltriethoxysilane | |
| 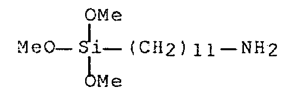 | 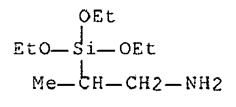 |
| 40762-31-0 11-Aminoundecyltrimethoxysilane | 36957-84-3 (2-Aminoisopropyl)triethoxysilane |
| | 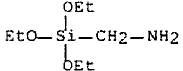 |
| | 18306-83-7 |
| | Aminomethyltriethoxysilane |
| | 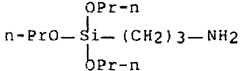 |
| | 18082-68-3 |
| | 1-Propanamine, 3-(tripropoxysilyl) |
| 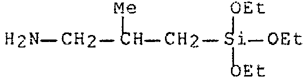 | |
| 17961-40-9 | |
| 1-Propanamine, 2-methyl-3-(triethoxysilyl)- | |
| | 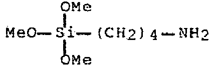 |
| | 15005-59-1 |
| | (4-Aminobutyl)trimethoxysilane |
| | 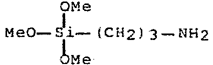 |
| | 13822-56-5 |
| | (3-Aminopropyl)trimethoxysilane |
| 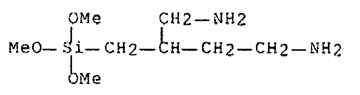 | 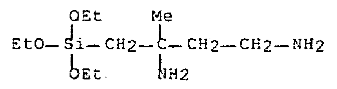 |
| 6037-49-6 1,4-Butanediamine, 2-[(trimethoxysilyl)methyl] | 5888-01-7 1,3-Butanediamine, 3-methyl-4-(triethoxysilyl) |
| 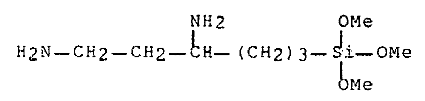 | 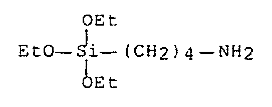 |
| 4543-14-0 1,3-Hexanediamine, 6-(trimethoxysilyl) | 3069-30-5 4-Aminobutyltriethoxysilane |

Selon un mode de réalisation très avantageux de l'invention, le composé de formule (I) est le (3-aminopropyl)triéthoxysilane.

Habituellement, la teneur en composés de formule (I) représente de 0,1 à 65 % en poids par rapport au poids total de la composition (B).

Selon un mode de réalisation particulier de l'invention, la teneur en composés de formule (I) représente de 0,1 à 50 % et de préférence de 1 à 30 % en poids par rapport au poids de la composition (B).

Selon un autre mode de réalisation particulier de l'invention, la teneur en composés de formule (I) représente de 20 à 65 %, encore plus préférentiellement de 30 à 60 %, mieux de 40 à 50 % en poids par rapport au poids de la composition (B).

Lorsque la composition (B) est aqueuse, le ou les composés organiques de silicium peuvent être partiellement neutralisés au moyen d'un agent de neutralisation ou régulateur de pH, de telle sorte que la neutralisation atteigne 1/1000 à 99/100 et mieux de 0,2/100 à 70/100. De préférence encore, la neutralisation est de 0,2/100 à 60/100.

Les agents régulateurs de pH peuvent être tous les acides ou mélanges d'acides cosmétiquement acceptables et solubles dans le milieu de la composition. Parmi les acides utilisables, on peut citer l'acide chlorhydrique, l'acide phosphorique, l'acide sulfonique et les acides organiques. La composition utilisée selon l'invention peut également contenir un ou plusieurs autres acides organiques.

Les acides organiques sont généralement choisis parmi les acides comportant une ou plusieurs fonctions acide carboxylique, sulfonique, phosphonique ou phosphorique. Ils peuvent contenir d'autres fonctions chimiques, en particulier des fonctions hydroxy ou amino. Ils peuvent être saturés ou insaturés. On peut citer en particulier l'acide acétique, l'acide propanoïque, l'acide butanoïque, l'acide lactique, l'acide glycolique, l'acide ascorbique, l'acide maléique, l'acide phtalique, l'acide succinique, la taurine, l'acide tartrique, l'acide gluconique, l'acide glucuronique et l'acide citrique. Les acides organiques préférés sont l'acide lactique, l'acide acétique, l'acide citrique.

La composition contenant le ou les composés organiques du silicium peut en plus contenir un ou plusieurs épaississants. Les agents épaississants peuvent être choisis parmi les amides d'acides gras (diéthanol- ou monoéthanol-amide de coprah, monoéthanolamide d'acide alkyl éther carboxylique oxyéthyléné), les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique.

Les compositions (A) et / ou (B) peuvent également comprendre :
- un ou plusieurs précurseurs de colorants d'oxydation, plus particulièrement une ou plusieurs bases d'oxydation éventuellement combinée(s) à un ou plusieurs coupleurs,
- un ou plusieurs colorants directs synthétiques,
- un ou plusieurs colorants naturels,
   ou leurs mélanges.

Selon un mode de réalisation particulier de l'invention, la composition (A) comprend également :
- un ou plusieurs précurseurs de colorants d'oxydation, plus particulièrement une ou plusieurs bases d'oxydation éventuellement combinée(s) à un ou plusieurs coupleurs,
- un ou plusieurs colorants directs synthétiques,
- un ou plusieurs colorants naturels,
   ou leurs mélanges.

Selon un autre mode de réalisation particulier de l'invention, la composition (B) comprend également :
- un ou plusieurs précurseurs de colorants d'oxydation, plus particulièrement une ou plusieurs bases d'oxydation éventuellement combinée(s) à un ou plusieurs coupleurs,
- un ou plusieurs colorants directs synthétiques,
- un ou plusieurs colorants naturels,
   ou leurs mélanges.

A titre d'exemple, les bases d'oxydation sont choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β -hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

A titre de dérivés pyrazoliques on peut également citer les diamino N,N-dihydropyrazolopyrazolones et notamment celles décrites dans la demande FR 2886136 telles que les composés suivants et leurs sels d'addition : 2,3-Diamino-6,7-dihydro-1 H,5H-pyrazolo[1,2-a]pyrazol-1-one, 2-Amino-3-éthylamino-6,7-dihydro-1H,5H-pyrazolo[1,2-a]pyrazol-1-one, 2-Amino-3-isopropylamino-6,7-dihydro-1H,5H-pyrazolo[1,2-a]pyrazol-1-one, 2-Amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-Amino-1,2-diethyl-5-(pyrrolidin-1-yl)-1,2-dihydro-pyrazol-3-one, 4-Amino-5-(3-dimethylamino-pyrrolidin-1 -yl)-1,2-diethyl-1,2-dihydro-pyrazol-3-one, 2,3-diamino-6-hydroxy-6,7-dihydro-1H,5H-pyrazolo[1,2-a]pyrazol-1-one.

A titre de bases hétérocycliques on utilisera préférentiellement le 4,5-diamino 1-(β-hydroxyéthyl) pyrazole et/ou la 2,3-diamino-6,7-dihydro-1H,5H-pyrazolo[1,2,A]pyrazol-1-one et leurs sels d'addition.

A titre de bases d'oxydation cationiques, on peut citer par exemple les composés suivants : les para-phénylènediamines telles que notamment décrites dans les demandes de brevets FR-A-2 766 177 et FR-A-2 766 178, les para-aminophénols tels que décrits par exemple dans les demandes de brevet FR-A-2 766 177 et FR-A-2 766 178, les ortho-phénylènediamines telles que décrites par exemple dans les demandes de brevet FR-A-2 782 718, FR-A-2 782 716 et FR-A-2 782 719, des ortho-aminophénols ou des bases doubles cationiques telles que des dérivés de type bis(aminophényl)alkylènediamine décrites dans les demandes de brevet FR-A-2 766 179, ainsi que les bases hétérocycliques cationiques, ces composés portant au moins un atome d'azote quaternaire.

De préférence, les bases d'oxydation cationiques sont des para-phénylènediamines cationiques.

De manière avantageuse, une variante consiste à mettre en oeuvre des bases d'oxydation cationiques de structure para-phénylènediamine, dont au moins une des fonctions amine est une amine tertiaire porteuse d'un noyau pyrrolidinique, la molécule possédant au moins un atome d'azote quaternisé. De telles bases sont, par exemple, décrites dans le document EP-A-1 348 695.

Les compositions selon l'invention peuvent éventuellement comprendre un ou plusieurs coupleurs choisis avantageusement parmi ceux conventionnellement utilisés pour la teinture des fibres kératiniques.

Parmi ces coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation, quand elles sont présentes dans la composition, représentent avantageusement de 0,0001 à 10 % en poids par rapport au poids de la composition, et de préférence de 0,005 à 5 % en poids par rapport au poids de la composition.

Le ou les coupleurs, s'ils sont présents, représentent avantageusement de 0,0001 à 10 % en poids par rapport au poids de la composition, et de préférence de 0,005 à 5 % en poids par rapport au poids de la composition.

A titre d'exemples de colorants directs synthétiques convenables, on peut citer les colorants directs azoïques ; méthiniques ; carbonyles ; aziniques ; nitrés (hétéro)aryle ; tri-(hétéro)aryle méthanes ; seuls ou en mélanges.

Plus particulièrement, les colorants azoïques comprennent une fonction -N=N- dont les deux atomes d'azote ne sont pas simultanément engagés dans un cycle. Il n'est toutefois pas exclu que l'un des deux atomes d'azote de l'enchaînement -N=N- soit engagé dans un cycle.

Les colorants de la famille des méthines sont plus particulièrement des composés comprenant au moins un enchaînement choisi parmi >C=C< et -N=C< dont les deux atomes ne sont pas simultanément engagés dans un cycle. Il est toutefois précisé que l'un des atomes d'azote ou de carbone des enchaînements peut être engagé dans un cycle. Plus particulièrement, les colorants de cette famille sont issus de composés de type méthine vraie (comprenant un ou plusieurs enchaînements -C=C- précités) ; azométhine (comprenant au moins un ou plusieurs enchaînements -C=N-) avec par exemple les azacabocyanines et leurs isomères, les diazacarbocyanines et leurs isomères, les tétraazacarbocyanines ; mono- et di- arylméthane ; indoamines (ou diphénylamines) ; indophénols ; indoanilines.

Concernant les colorants de la famille des carbonyles, on peut citer par exemple les colorants choisis parmi les acridone, benzoquinone, anthraquinone, naphtoquinone, benzanthrone, anthranthrone, pyranthrone, pyrazolanthrone, pyrimidinoanthrone, flavanthrone, idanthrone, flavone, (iso)violanthrone, isoindolinone, benzimidazolone, isoquinolinone, anthrapyridone, pyrazoloquinazolone, périnone, quinacridone, quinophthalone, indigoïde, thioindigo, naphtalimide, anthrapyrimidine, dicétopyrrolopyrrole, coumarine.

Concernant les colorants de la famille des aziniques, on peut citer notamment les azine, xanthène, thioxanthène, fluorindine, acridine, (di)oxazine, (di)thiazine, pyronine.

Les colorants nitrés (hétéro)aromatiques sont plus particulièrement des colorants directs nitrés benzéniques ou nitrés pyridiniques.

Concernant les colorants de type porphyrines ou phtalocyanines, on peut mettre en oeuvre des composés cationiques ou non, comprenant éventuellement un ou plusieurs métaux ou ions métalliques, comme par exemple des métaux alcalins et alcalino-terreux, le zinc et le silicium.

A titre d'exemples de colorants directs de synthèse particulièrement convenables, on peut citer les colorants nitrés de la série benzénique ; les colorants directs azoïques ; méthiniques ; azométhiniques avec plus particulièrement les diazacarbocyanines et leurs isomères et les tétraazacarbocyanines (tétraazapentaméthines) ; les colorants directs quinoniques et en particulier anthraquinoniques, naphtoquinoniques ou benzoquinoniques ; les colorants directs aziniques ; xanthéniques ; triarylméthaniques ; indoaminiques ; indigoïdes ; phtalocyanines et porphyrines ; seuls ou en mélanges.

De préférence, les colorants directs sont choisis parmi les colorants nitrés de la série benzénique ; azoïques ; azométhines avec les diazacarbocyanines et leurs isomères, les tétraazacarbocyanines (tétraazapentaméthines) ; les colorants directs anthraquinoniques ; les colorants directs triarylméthaniques ; seuls ou en mélanges.

De manière encore plus préférée, ces colorants directs sont choisis parmi les colorants nitrés de la série benzénique ; les colorants directs azoïques ; azométhines avec les diazacarbocyanines et leurs isomères, les tétraazacarbocyanines (tétraazapentaméthines) ; seuls ou en mélange.

Ces colorants peuvent être des colorants monochromophoriques (c'est-à-dire ne comprenant qu'un seul colorant) ou polychromophoriques, de préférence di- ou tri-chromophoriques ; les chromophores pouvant être identiques ou non, de la même famille chimique ou non. A noter que qu'un colorant polychromophorique comprend plusieurs radicaux issus chacun d'une molécule absorbant dans le domaine visible entre 400 et 800 nm. De plus cette absorbance du colorant ne nécessite ni oxydation préalable de celui-ci, ni association avec d'autre(s) espèce(s) chimique(s).

Dans le cas de colorants polychromophoriques, les chromophores sont reliés entre eux au moyen d'au moins un bras de liaison qui peut être cationique ou non.

Parmi les colorants directs nitrés benzéniques utilisables selon l'invention, on peut citer de manière non limitative les composés suivants: -1,4-diamino-2-nitrobenzène ; -1-amino-2 nitro-4-β- hydroxyéthylaminobenzène ; -1-amino-2 nitro-4-bis(β-hydroxyéthyl)-aminobenzène ; -1,4-Bis(β-hydroxyéthylamino)-2-nitrobenzène ; -1-β-hydroxyéthylamino-2-nitro-4-bis-(β-hydroxyéthylamino)-benzène ; -1-β-hydroxyéthylamino-2-nitro-4-aminobenzène ; -1-β-hydroxyéthylamino-2-nitro-4-(éthyl)(β-hydroxyéthyl)-aminobenzène ; -1-amino-3-méthyl-4-β-hydroxyéthylamino-6-nitrobenzène ; -1-amino-2-nitro-4-β-hydroxyéthylamino-5-chlorobenzène ; -1,2-Diamino-4-nitrobenzène ; -1-amino-2-β-hydroxyéthylamino-5-nitrobenzène ; -1,2-Bis-(β-hydroxyéthylamino)-4-nitrobenzène ; -1-amino-2-tris-(hydroxyméthyl)-méthylamino-5-nitrobenzène ; -1-Hydroxy-2-amino-5-nitrobenzène ; -1-Hydroxy-2-amino-4-nitrobenzène ; -1-Hydroxy-3-nitro-4-aminobenzène ; -1-Hydroxy-2-amino-4,6-dinitrobenzène ;-1-β-hydroxyéthyloxy-2-β-hydroxyéthylamino-5-nitrobenzène ;-1-Méthoxy-2-β-hydroxyéthylamino-5-nitrobenzène; -1-β-hydroxyéthyloxy-3-méthylamino-4-nitrobenzène ; -1-βγ-dihydroxypropyloxy-3-méthylamino-4-nitrobenzène ; -1-β-hydroxyéthylamino-4-β,γ-dihydroxypropyloxy-2-nitrobenzène ; -1-βγ-dihydroxypropylamino-4-trifluorométhyl-2-nitrobenzène ; -1-p-hydroxyéthylamino-4-trifluorométhyl-2-nitrobenzène ; -1-β-hydroxyéthylamino-3-méthyl-2-nitrobenzène ; -1-β-aminoéthylamino-5-méthoxy-2-nitrobenzène ; -1-Hydroxy-2-chloro-6-éthylamino-4-nitrobenzène ; -1-Hydroxy-2-chloro-6-amino-4-nitrobenzène ; -1-Hydroxy-6-bis-(β-hydroxyéthyl)-amino-3-nitrobenzène ; -1-β-hydroxyéthylamino-2-nitrobenzène ; -1-Hydroxy-4-β-hydroxyéthylamino-3-nitrobenzène.

Parmi les colorants directs monochromophoriques azoïques, azométhines, méthines utilisables selon l'invention on peut citer les colorants cationiques décrits dans les demandes de brevets WO 95/15144, WO 95/01772 et EP 714954 ; FR 2189006, FR 2285851, FR-2140205, EP 1378544, EP 1674073.

Ainsi, on peut tout notamment citer les colorants directs cationiques correspondants aux formules suivantes : 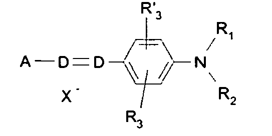 dans laquelle :
D représente un atome d'azote ou le groupement -CH,
R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₃ et R'₃, identiques ou différents, représentent un atome d'hydrogène ou d'halogène choisi parmi le chlore, le brome, l'iode et le fluor, un radical cyano, alkyl en C₁-C₄, alcoxy en C₁-C₄ ou acétyloxy,
X - représente un anion de préférence choisi parmi le chlorure, le méthyl sulfate et l'acétate,
A représente un groupement choisi par les structures suivantes : 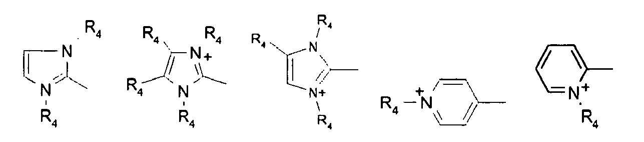 dans lesquelles R₄ représente un radical alkyle en C₁-C₄ pouvant être substitué par un radical hydroxyle ; 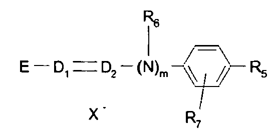 dans lesquelles :
R₅ représente un atome d'hydrogène, un radical alcoxy en C₁-C₄, un atome d'halogène tel que le brome, le chlore, l'iode ou le fluor,
R₆ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
R₇ représente un atome d'hydrogène ou d'halogène tel que le brome, le chlore, l'iode ou le fluor,
D₁ et D₂, identiques ou différents, représentent un atome d'azote ou le groupement -CH, m = 0 ou 1,
X - représente un anion cosmétiquement acceptable et de préférence choisi parmi le chlorure, le méthyl sulfate et l'acétate,
E représente un groupement choisi par les structures suivantes : 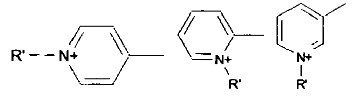 dans lesquelles R' représente un radical alkyle en C₁-C₄ ;
lorsque m = 0 et que D₁ représente un atome d'azote, alors E peut également désigner un groupement de structure suivante : 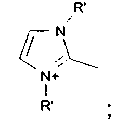 dans laquelle R' représente un radical alkyle en C₁-C₄.

Parmi les composés précités, on utilise tout particulièrement les composés suivants :

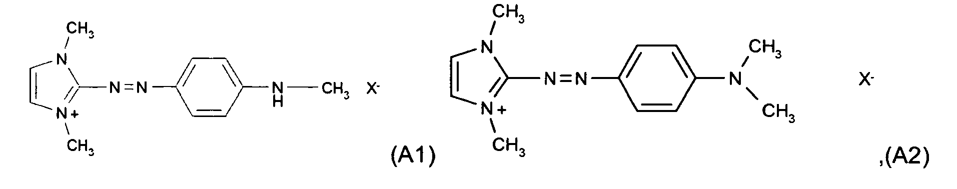

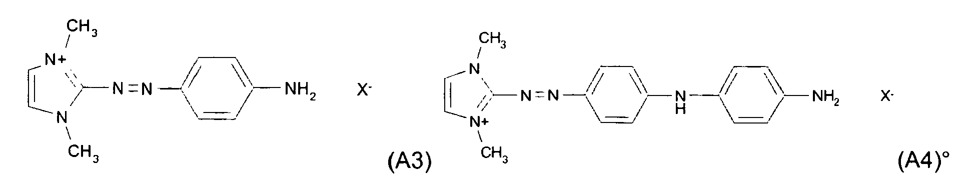

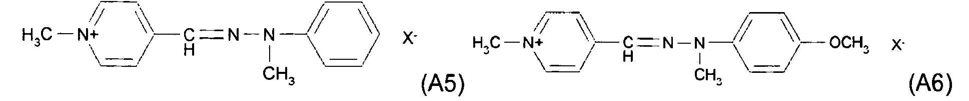

Parmi les colorants de type tétraazapentaméthiniques utilisables selon l'invention, on peut citer les composés suivants figurant dans le tableau ci-dessous :

| | |
|---|---|
| 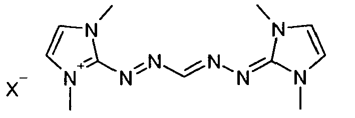 | 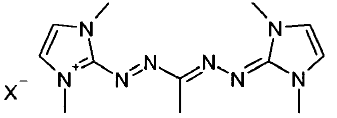 |
| 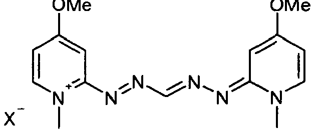 | 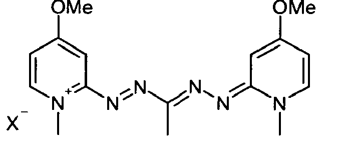 |
| 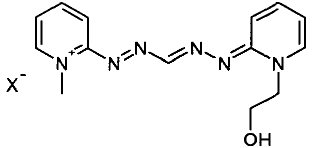 | 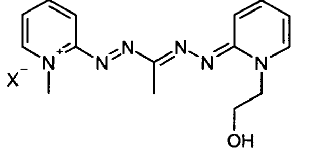 |
| 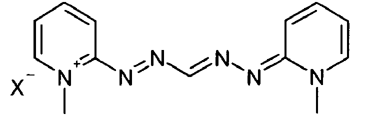 | 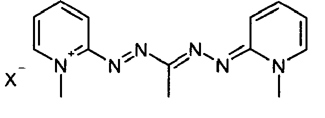 |
| 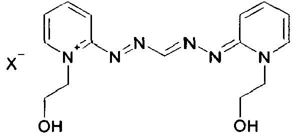 | 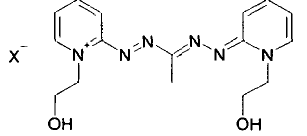 |

X⁻ représente un anion de préférence choisi parmi le chlorure, l'iodure, le méthyl sulfate, l'éthyl sulfate, l'acétate.

Comme autres colorants utilisables selon l'invention on peut également citer parmi les colorants directs azoïques les colorants suivants, décrits dans le COLOUR INDEX INTERNATIONAL 3e édition : Disperse Red 17 ; -Disperse Red 13 ; Basic Red 22 ; Basic Red 76 ; Basic Yellow 57 ; Basic Brown 16 ; Basic Brown 17 ; Disperse Green 9 ; Disperse Black 9 ; Solvent Black 3 ; Disperse Blue 148 ; Disperse Violet 63 ; Solvent Orange 7 ; 1-(4'-aminodiphénylazo)-2-méthyl-4bis-(β-hydroxyéthyl) aminobenzène(Nom INCI :HC Yellow 7).

Parmi les colorants directs quinoniques on peut citer les colorants suivants : Disperse Red 15 ; Solvent Violet 13 ; Solvent Blue 14 ; Disperse Violet 1 ; Disperse Violet 4 ; Disperse Blue 1 ; Disperse Violet 8 ; Disperse Blue 3 ; Disperse Red 11 ; Disperse Blue 7 ; Disperse Blue 14 ; Basic Blue 22 ; Disperse Violet 15 ; Disperse Blue 377 ; Disperse Blue 60 ; Basic Blue 99. On peut également citer les composés suivants : 1-N-méthylmorpholiniumpropylamino-4-hydroxyanthraquinone ; 1-Aminopropylamino-4-méthyl aminoanthraquinone ; -1-Aminopropylaminoanthraquinone ; 5-β-hydroxyéthyl-1,4-diamino anthraquinone ; 2-Aminoéthylaminoanthraquinone ; 1,4-Bis-(βγ-dihydroxypropyl amino)-anthraquinone, ainsi que la coumarine Disperse Yellow 82.

Parmi les colorants aziniques on peut citer les composés suivants : Basic Blue 17 ; Basic Red 2 ; Solvent Orange 15.

Parmi les colorants triarylméthaniques utilisables selon l'invention, on peut citer les composés suivants : Basic Green 1 ; Basic Violet 3 ; Basic Violet 14 ; Basic Blue 7 ; Basic Blue 26.

Parmi les colorants indoaminiques utilisables selon l'invention, on peut citer les composés suivants : 2-β-hydroxyéthylamino-5-[bis-(β-4'-hydroxyéthyl)amino]anilino-1,4-benzoquinone ; 2-β-hydroxyéthylamino-5-(2'-méthoxy-4'-amino)anilino-1,4-benzo quinone ; 3-N(2'-Chloro-4'-hydroxy)phényl-acétylamino-6-méthoxy-1,4-benzoquinone imine ; -3-N(3'-Chloro-4'-méthylamino)phényl-uréido-6-méthyl-1,4-benzoquinone imine ; 3-[4'-N-(Ethyl,carbamylméthyl)-amino]-phényl-uréido-6-méthyl-1,4-benzoquinone imine.

De préférence, les colorants directs cationiques sont choisis parmi les colorants directs monochromophoriques de types azoïques ; méthines vraies ; azométhines avec les diazacarbocyanines et leurs isomères, les tétraazacarbocyanines (tétraazapentaméthines) ; anthraquinoniques ; seuls ou en mélange.

S'ils sont présents dans les compositions (A) et / ou (B), la teneur en colorant(s) direct(s) représente(nt) de 0,005 à 20 % et de préférence de 0,01 à 10 %, mieux de 0,05 à 5 % en poids par rapport au poids de ladite composition.

Les compositions (A) et / ou (B) peuvent également comprendre un ou plusieurs colorants naturels de préférence choisis parmi la lawsone, la juglone, l'alizarine, la purpurine, l'acide carminique, l'acide kermésique, la purpurogalline, l'isatine, l'indigo, le protocatéchaldéhyde, les anthocyanes et anthocyanidines, la curcumine, les orcéines, l'apigéninidine. On peut utiliser ces composés tels quels ou à partir d'extraits.

S'ils sont présents dans les compositions (A) et / ou (B), la teneur en colorant(s) naturels (s) représente(nt) de 0,005 à 20 % et de préférence de 0,01 à 10 %, mieux de 0,05 à 5 % en poids par rapport au poids de ladite composition.

La composition (B) peut encore comprendre un agent alcalin différent des composés de formule (I) et pouvant être choisi parmi l'ammoniaque ou parmi ceux décrits dans le cadre de la composition (A).

De préférence, l'agent alcalin est choisi parmi les alcanolamines, les acides aminés basiques et les hydroxydes ou les carbonates de métaux alcalins.

Plus particulièrement, l'agent alcalin est choisi parmi les alcanolamines éventuellement en mélange avec les acides aminés basiques, les hydroxydes ou les carbonates de métaux alcalins.

Selon un mode de réalisation particulièrement avantageux de l'invention, l'agent alcalin est la monoéthanolamine utilisée seule ou en mélange avec les agents alcalins précités, en particulier avec une base minérale telle que par exemple l'hydroxyde de sodium ou le carbonate de potassium, et/ou encore avec un acide aminé basique comme en particulier l'arginine.

La composition (B) comprend une teneur en agent(s) alcalin(s) s'il est ou s'ils sont présents comprise entre 0,1 et 30 % en poids par rapport au poids de ladite composition, de préférence entre 0,5 et 25 %, mieux entre 1 et 20 % en poids.

Cet agent, ainsi que les agents régulateurs de pH définis précédemment, peuvent permettre de régler le pH de la composition appliquée sur cheveux.

Selon un mode de réalisation particulier de l'invention, le pH de la composition appliquée sur cheveux est compris entre 4 et 11 et encore plus préférentiellement entre 7 et 10,5.

Selon un autre mode de réalisation particulier de l'invention, le pH de la composition prête à l'emploi est compris entre 3 et 12 environ, de préférence entre 5 et 11 environ.

La composition comprenant le ou les composés organiques du silicium présente généralement un pH compris entre 2 et 13, de préférence entre 4 et 11. Plus préférentiellement, le pH de cette composition obtenue avec l'agent régulateur de pH est compris entre 7 et 10,5, encore plus préférentiellement, le pH est compris entre 8 et 10.

Si l'ammoniaque est employée en tant qu'agent alcalinisant supplémentaire, alors de préférence, sa teneur est inférieure ou égale à 0,03 % en poids de la composition finale (exprimée en NH₃), plus particulièrement inférieure ou égale à 0,01 % en poids par rapport à la composition finale. Il est rappelé que la composition finale résulte du mélange des trois compositions (A), (B) et (C) ; ce mélange étant réalisé de préférence avant application sur les fibres kératiniques (préparation extemporanée).

Mais de manière particulièrement avantageuse, la composition (B) ne comprend pas d'ammoniaque.

La composition (B) peut également renfermer divers adjuvants utilisés classiquement dans les compositions pour la coloration des cheveux, tels que des polymères anioniques, cationiques, non ioniques, amphotères, zwitterioniques ou leurs mélanges ; des agents épaississants minéraux, et en particulier des charges telles que des argiles, le talc ; des agents épaississants organiques, avec en particulier les épaississants associatifs polymères anioniques, cationiques, non ioniques et amphotères ; des agents antioxydants ; des agents de pénétration ; des agents séquestrants ; des parfums ; des agents dispersants ; des agents filmogènes ; des céramides ; des agents conservateurs ; des agents opacifiants ; des agents conditionneurs avec en particuliers des polymères cationiques.

Les adjuvants ci dessus sont en général présents en quantité comprise pour chacun d'eux entre 0,01 et 20 % en poids par rapport au poids de la composition.

Enfin, la composition (C) mise en oeuvre dans le procédé selon l'invention comprend un ou plusieurs agents oxydants (composition oxydante).

Elle est avantageusement une émulsion directe huile dans eau.

Plus particulièrement, la composition oxydante est aqueuse et comprend éventuellement un ou plusieurs solvants organiques.

A titre de solvant organique, on peut par exemple citer, les alcanols, linéaires ou ramifiés, en C₂-C₄, tels que l'éthanol et l'isopropanol ; le glycérol ; les glycols et éthers de glycols comme le 2-butoxyéthanol, le propylèneglycol, le dipropylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Le ou les solvants peuvent être présents dans des proportions allant habituellement de 1 à 40 % en poids par rapport au poids de la composition oxydante, et de préférence de 5 à 30 % en poids.

Plus particulièrement, l'agent oxydant est choisi parmi le peroxyde d'hydrogène ; le peroxyde d'urée ; les bromates ou ferricyanures de métaux alcalins ; les sels peroxygénés comme par exemple les persulfates, les perborates et les percarbonates de métaux alcalins ou alcalino-terreux, comme le sodium, le potassium, le magnésium ; ou leurs mélanges. On peut également utiliser à titre d'agent oxydant une ou plusieurs enzyme(s) d'oxydo-réduction telles que les laccases, les peroxydases et les oxydoréductases à 2 électrons (telles que l'uricase), éventuellement en présence de leur donneur ou cofacteur respectif.

Cet agent oxydant est avantageusement constitué du peroxyde d'hydrogène et notamment par une solution aqueuse dont le titre peut varier, plus particulièrement, de 1 à 40 volumes, et encore plus préférentiellement de 5 à 40 volumes.

La composition oxydante peut également comprendre au moins un agent alcalinisant et/ou au moins un agent acidifiant. De préférence la composition oxydante contient au moins un agent acidifiant.

Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

De préférence, le pH de la composition oxydante est inférieur à 7 en particulier si l'agent oxydant est le peroxyde d'hydrogène.

La composition oxydante peut se présenter sous la forme d'une solution, une émulsion ou un gel.

La teneur en eau varie de préférence de 5 à 95, % en poids, encore plus préférentiellement de 25 à 92 % en poids, mieux de 40 à 90% en poids, par rapport au poids de la composition (C).

De préférence, la teneur en eau de la composition (C) comprise entre 10 et 90 % en poids, par rapport au poids de l'émulsion.

Lorsque la composition (C) est une émulsion huile dans eau, elle comprend un ou plusieurs corps gras tel que décrit dans le cadre de la composition (A) ; partie à laquelle on pourra se reporter.

L'émulsion (C) selon l'invention comprend de préférence au moins 10 % de corps gras. De préférence la concentration en corps gras va de 10 à 80 %, encore plus préférentiellement de 15 à 65 %, mieux de 20 à 55 % du poids total de l'émulsion.

Selon un mode de réalisation particulier, l'émulsion contient une ou plusieurs huiles. A titre d'exemple, on peut citer l'huile de vaseline, l'huile de paraffine, les polydécènes, les esters d'acides gras ou d'alcools gras liquides.

L'émulsion (C) comprend également un ou plusieurs tensioactifs.

De préférence, le ou les tensioactifs sont choisis parmi les tensioactifs non ioniques ou parmi les tensioactifs anioniques, de préférence non ioniques.

De préférence, le tensioactif présent dans l'émulsion est un tensioactif non ionique oxyéthyléné présentant une HLB de 8 à 18. Le HLB est le rapport entre la partie hydrophile et la partie lipophile dans leur molécule. Ce terme HLB est bien connu de l'homme du métier et est décrit dans "The HLB system. A time-saving guide to Emulsifier Selection" (published by ICI Americas Inc; 1984).

Dans une variante préférée la composition (C) ne contient pas de tensioactifs glycérolés.

La teneur en tensioactifs dans l'émulsion (C) représente plus particulièrement de 0,1 à 50 % en poids, de préférence de 0,5 à 30 % en poids par rapport au poids de l'émulsion.

Selon un mode de réalisation particulier, lorsque la composition (C) est une émulsion directe, elle peut être préparée par des procédés de préparation classique d'émulsion directe mais aussi par un procédé par PIT. De préférence l'émulsion directe oxydante est préparée par un procédé PIT.

Selon ce mode de réalisation particulier, le principe d'émulsification par inversion de phase en température (en Anglais : Phase Inversion Temperature ou PIT) est, dans son principe, bien connu de l'homme de l'art ; il a été décrit en 1968 par K. Shinoda (J. Chem. Soc. Jpn., 1968, 89, 435). Il a été montré que cette technique d'émulsification permet d'obtenir des émulsions fines stables (K. Shinoda et H. Saito, J. Colloïd Interface Sci., 1969,30, 258). Cette technologie a été appliquée en cosmétique dès 1972 par Mitsui et al. («Application of the phase-inversion-temperature method to the emulsification of cosmetics» ; T. Mitsui, Y. Machida and F. Harusawa, American. Cosmet. Perfum., 1972, 87, 33).

Le principe de cette technique est le suivant : on réalise le mélange d'une phase aqueuse et d'une phase huileuse, que l'on porte à une température supérieure à la température PIT, température d'inversion de phase du système, qui est la température à laquelle l'équilibre entre les propriétés hydrophile et lipophile du ou des émulsionnants mis en oeuvre est atteint ; à température élevée, c'est-à-dire supérieure à la température d'inversion de phase (>PIT), l'émulsion est de type eau-dans-huile, et, au cours de son refroidissement, cette émulsion s'inverse à la température d'inversion de phase, pour devenir une émulsion de type huile-dans-eau, et ceci en étant passée auparavant par un état de microémulsion. Ce procédé permet d'obtenir facilement des émulsions de diamètre inférieur à 4 µm.

Selon ce procédé PIT, l'émulsion directe (C) comprend une émulsion directe (huile dans eau) qui comprend au moins 25 % d'un ou plusieurs corps gras dont au moins une huile, un ou plusieurs tensioactifs dont au moins un est un tensioactif non ionique présentant un point de trouble, une quantité d'eau supérieure à 5 % en poids, du poids total de l'émulsion. Selon ce mode de réalisation particulier, le tensioactif non ionique présente un HLB compris entre 8 et 18. Il est de préférence choisi parmi les tensioactifs oxyalkylénés, de préférence oxyéthylénés tels que les alcools gras éthoxylés, les acides gras éthoxylés, les glycérides partiels d'acides gras éthoxylés, les triglycérides d'acides gras polyglycérolés et leurs dérivés éthoxylés, et leurs mélanges. Par ailleurs, une telle émulsion présente une taille de particule inférieure à 4 µm, de préférence inférieure à 1µm.

De manière plus détaillée, on peut opérer de manière suivante pour obtenir une émulsion PIT :
1) Peser dans un récipient tous les constituants de l'émulsion directe (C)
2) Homogénéiser le mélange, par exemple au moyen d'un Rayneri 350 tours/min, et chauffer en augmentant progressivement la température au moyen d'un bain marie jusqu'à une température supérieure à la température d'inversion de phase T1, c'est-à-dire jusqu'à l'obtention d'une phase transparente ou translucide (zone de microémulsion ou de phase lamellaire) puis d'une phase plus visqueuse qui indique l'obtention de l'émulsion inverse (E/H).
3) Arrêter le chauffage et maintenir l'agitation jusqu'à retour à la température ambiante, en passant par la température d'inversion de phase T1, c'est-à-dire la température à laquelle se forme une émulsion H/E fine.
4) Lorsque la température est redescendue en dessous de la zone d'inversion de Phase en Température (T1), additionner les éventuels additifs et les matières premières thermosensibles.

On obtient une composition finale stable dont les gouttelettes de phase lipophile sont fines avec des tailles de 10 à 200 nm.

Dans la zone de formation d'une microémulsion (mélange translucide), les interactions hydrophiles et hydrophobes sont équilibrées car la tendance du tensioactif est de former aussi bien des micelles directes que des micelles inverses. Par chauffage au-delà de cette zone, il y a formation d'une émulsion E/H, car le tensioactif favorise la formation d'une émulsion eau dans huile. Puis lors du refroidissement en dessous de la zone d'inversion de phase, l'émulsion devient une émulsion directe (H/E).

L'émulsification par inversion de phase est expliquée en détail dans l'ouvrage T.Fôrster, W von Rybinski, A.Wadle, Influence of microemulsion phases on the preparation of fine disperse emulsions, Advances in Colloid and interface sciences, 58, 119-149, 1995 cité ici pour référence.

La composition oxydante peut de même renfermer d'autres ingrédients classiquement employés dans le domaine, comme notamment ceux détaillés auparavant dans le cadre des compositions (A) et (B).

Selon un mode de réalisation particulier de l'invention, la quantité de composition oxydante par rapport à celle des compositions (A) et (B), est telle que la teneur en composé(s) de formule (I) soit comprise entre 2 et 8 % en poids dans la composition finale ; c'est-à-dire de la composition résultant du mélange des compositions (A), (B) et (C) ; ce mélange étant réalisé de préférence avant application sur les fibres kératiniques (préparation extemporanée).

La composition oxydante peut également, comprendre des adjuvants tels que ceux décrits dans le cadre des définitions des compositions (A) et (B), dans les gammes de teneurs également indiquées.

Selon un mode de réalisation particulier, la composition contenant le ou les précurseurs de colorant avant mélange et la composition finale prête à l'emploi avec oxydant comprennent un ou plusieurs polymères cationiques dont la densité de charge cationique est supérieure ou égale à 4 milliéquivalents par gramme (meq/g), de préférence supérieure ou égale à 5 milliéquivalents par gramme (meq/g), de préférence allant de 5 à 20 méq/g et plus particulièrement de 5,5 à 10 meq/g.

La densité de charge cationique d'un polymère correspond au nombre de moles de charges cationiques par unité de masse de polymère dans les conditions où celui-ci est totalement ionisé. Elle peut être déterminée par calcul si on connaît la structure du polymère, c'est-à-dire la structure des monomères constituant le polymère et leur proportion molaire ou pondérale. Elle peut être aussi déterminée expérimentalement par la méthode Kjeldahl, généralement à un pH d'environ 7 à température ambiante.

Les polymères cationiques ayant une densité de charge cationique supérieure à 4 méq/g, peuvent être choisis parmi tous ceux déjà connus en soi comme améliorant les propriétés cosmétiques des cheveux traités par des compositions, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière générale, au sens de la présente invention, l'expression "polymère cationique" désigne tout polymère comprenant des groupes cationiques et/ou des groupes ionisables en groupes cationiques.

Les polymères cationiques sont choisis parmi ceux qui comprennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse molaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Parmi ces polymères, on peut citer :
(1) les homopolymères ou copolymères dérivés d'esters ou d'amides acryliques ou méthacryliques et comportant au moins un des motifs de formules suivantes: 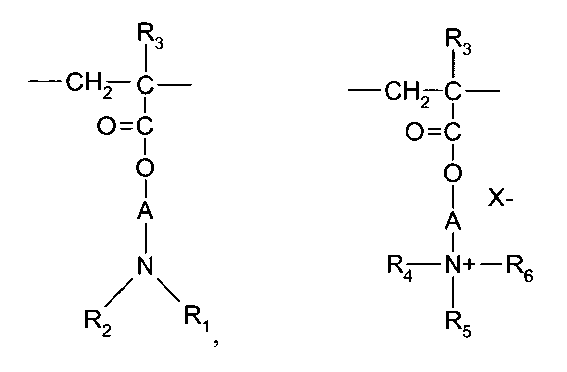 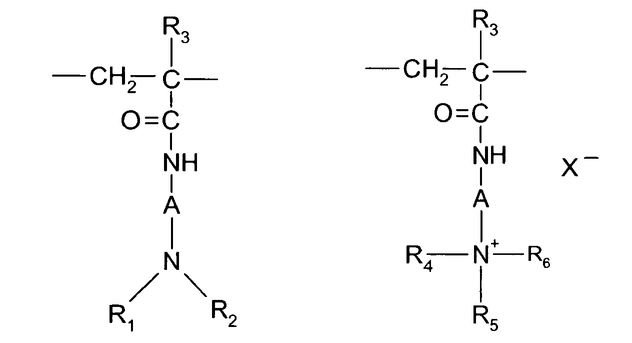 dans lesquelles:
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique en particulier un anion méthosulfate ou un halogénure, notamment chlorure ou bromure.
   Les copolymères de la famille (1) peuvent comprendre en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.

Ainsi, parmi ces copolymères de la famille (1), on peut citer :
- les copolymères d'acrylamide et de diméthylaminoéthyl méthacrylate quaternisé au sulfate de diméthyle ou avec un halogénure de diméthyle
- les copolymères d'acrylamide et de chlorure de méthacryloyloxyéthyltriméthyl-ammonium décrit par exemple dans la demande de brevet EP-A-080976
- le copolymères d'acrylamide et de méthosulfate de méthacryloyloxyéthyltriméthyl-ammonium,
- les copolymères vinylpyrrolidone / acrylate ou méthacrylate de dialkylaminoalkyle quaternisés ou non. Ces polymères sont décrits en détail dans les brevets français 2.077.143 et 2.393.573,
- les terpolymères méthacrylate de diméthyl amino éthyle/ vinylcaprolactame/vinylpyrrolidone,
- les copolymères vinylpyrrolidone / méthacrylamidopropyl dimethylamine.
- les copolymères vinylpyrrolidone / méthacrylamide de diméthylaminopropyle quaternisé,
- et les polymères réticulés de sels de méthacryloyloxyalkyl(C1-C4) trialkyl(C1-C4)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion comprenant 50 % en poids dudit copolymère dans de l'huile minérale. Cette dispersion est commercialisée sous le nom de « SALCARE® SC 92 » par la Société CIBA. On peut également utiliser un homopolymère réticulé du chlorure de méthacryloyloxyéthyl triméthylammonium comprenant environ 50 % en poids de l'homopolymère dans de l'huile minérale ou dans un ester liquide. Ces dispersions sont commercialisées sous les noms de « SALCARE® SC 95 » et « SALCARE® SC 96 » par la Société CIBA.

(2) les cyclopolymères d'alkyl diallyl amine ou de dialkyl diallyl ammonium tels que les homopolymères ou copolymères comportant comme constituant principal de la chaîne des motifs répondant aux formules (VIII) ou (IX) : 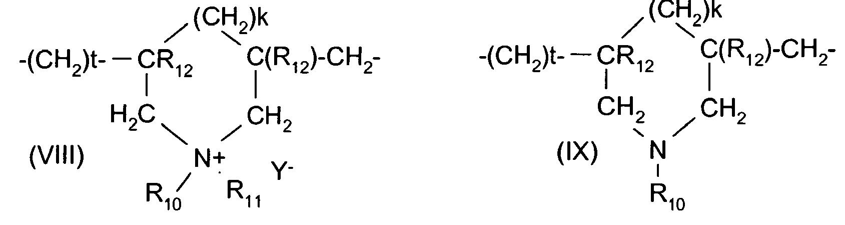 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄) ou R₁₀ et R₁₁ peuvent désigner conjointement avec l'atome d'azote auquel ils sont rattachés, des groupement hétérocycliques, tels que pipéridinyle ou morpholinyle ; Y- est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.

Parmi les polymères définis ci-dessus, on peut citer plus particulièrement les homopolymères de sels (par exemple chlorure) de diméthyldiallylammonium vendus notamment sous la dénomination "MERQUAT 100" par la société NALCO (et ses homologues de faibles masses molaires moyenne en poids) et les copolymères de chlorure de diallyldiméthylammonium et d'acrylamide.
(3) Les copolymères quaternaires de vinyllactame (vinylpyrrolidone et/ou vinylcaprolactame) et de vinylimidazole
(4) le polymère de diammonium quaternaire contenant des motifs récurrents répondant à la formule : 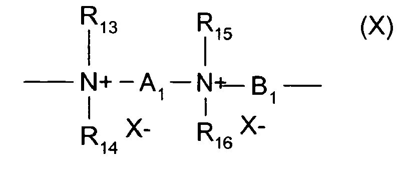 formule (X) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkyl aliphatiques inférieurs en C₁-C₆, ou bien R₁₃, R₁₄, R₁₅ et R₁₆, ensemble ou séparément, constituent avec les atomes d'azote auxquels ils sont rattachés des hétérocycles comprenant éventuellement un second hétéroatome autre que l'azote ou bien R₁₃, R₁₄, R₁₅ et R₁₆ représentent un radical alkyle en C₁-C₆ linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A₁ et B₁ représentent des groupements polyméthyléniques comprenant de 2 à 20 atomes de carbone pouvant être linéaires ou ramifiés, saturés ou insaturés, et pouvant comprendre, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- désigne un anion dérivé d'un acide minéral ou organique;
   A₁, R₁₃ et R₁₅ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B₁ peut également désigner un groupement (CH²)n-CO-D-OC-(CH₂)ₚ-
   dans lequel
   n et p, identiques ou différents, sont des nombres entiers variant de 2 à 20 environ D désigne :
   a) un reste de glycol de formule : -O-Z-O-, où Z désigne un radical hydrocarboné linéaire ou ramifié ou un groupement répondant à l'une des formules suivantes :

      -(CH₂-CH₂-O)ₓ-CH₂-CH₂-

      -[CH₂-CH(CH₃)-O]_{y}-CH₂-CH(CH₃)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH₂-CH₂-S-S-CH₂-CH₂- ;
   d) un groupement uréylène de formule : -NH-CO-NH-.

De préférence, X- est un anion tel que le chlorure ou le bromure.

Ces polymères ont une masse moléculaire moyenne en nombre généralement comprise entre 1000 et 100000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont essentiellement constitués de motifs récurrents répondant à la formule : 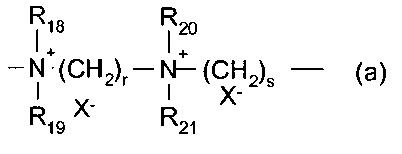 dans laquelle R₁₈, R₁₉, R₂₀ et R₂₁, identiques ou différents, désignent un radical alkyle ou hydroxyalkyle ayant de 1 à 4 atomes de carbone environ, r et s sont des nombres entiers variant de 2 à 20 environ et, X- est un anion dérivé d'un acide minéral ou organique.

Un composé de formule (a) particulièrement préféré est celui pour lequel R₁₈, R₁₉, R₂₀ et R₂₁, représentent un radical méthyle et r = 3, s = 6 et X = Cl, dénommé Hexadimethrine chloride selon la nomenclature INCI (CTFA).
(5) les polymères de polyammonium quaternaires constitués de motifs de formule (XI): 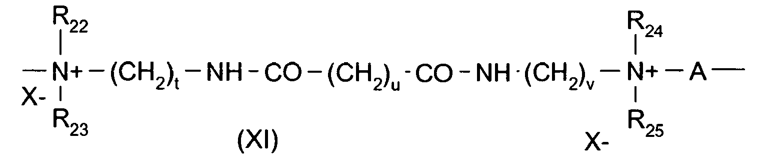 formule dans laquelle :
   R₂₂, R₂₃, R₂₄ et R₂₅, identiques ou différents, représentent un atome d'hydrogène ou un radical méthyle, éthyle, propyle, β-hydroxyéthyle, β-hydroxypropyle ou - CH₂CH₂(OCH₂CH₂)ₚOH,
   où p est égal à 0 ou à un nombre entier compris entre 1 et 6, sous réserve que R₂₂, R₂₃, R₂₄ et R₂₅ ne représentent pas simultanément un atome d'hydrogène,
   t et u, identiques ou différents, sont des nombres entiers compris entre 1 et 6,
   v est égal à 0 ou à un nombre entier compris entre 1 et 34,
   X- désigne un anion tel qu'un halogènure,
   A désigne un radical d'un dihalogénure ou représente de préférence -CH₂-CH₂-O-CH₂-CH₂-.
De tels composés sont notamment décrits dans la demande de brevet EP-A-122 324.

On peut par exemple citer parmi ceux-ci, les produits "Mirapol® A 15", "Mirapol® AD1", "Mirapol® AZ1" et "Mirapol® 175" vendus par la société Miranol.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les cyclopolymères cationiques, en particulier les homopolymères de chlorure de diméthyldiallylammonium, vendus sous les dénominations « MERQUAT 100 » par la société NALCO (et ses homologues de faibles masses molaires moyenne en poids), les polyéthylèneimines et leurs mélanges.

Selon l'invention, le ou les polymères cationiques ayant une densité cationique supérieure ou égale à 4meq/g peuvent représenter de 0,001 % à 10 % en poids, de préférence de 0,005 % à 5 % en poids, et encore plus préférentiellement de 0,01 % à 3 % en poids, du poids total de la composition finale prête à l'emploi ou de la composition comprenant le ou les précurseurs de colorant avant mélange.

Selon un autre mode de réalisation, la composition contenant le ou les précurseurs de colorant avant mélange et la composition finale prête à l'emploi avec oxydant comprennent en outre un ou plusieurs tensioactifs oxyalkylénés ou glycérolés non ioniques tels que définis précédemment.

Conformément à un mode de réalisation particulièrement avantageux de l'invention, la composition comprend au moins un tensioactif non ionique choisi parmi les alcools en C₆-C₃₀ oxyalkylénés ou glycérolés.

Selon le mode de réalisation particulier de l'invention décrit ci-dessus, la teneur totale en tensioactifs non ioniques oxyalkylénés ou glycérolés représente de 0,01 % à 50 % en poids, de préférence de 0,1 à 30 % en poids, mieux de 0,1 à 20 % et encore mieux de 0,1 à 10 % en poids par rapport au poids de la composition finale prête à l'emploi ou de la composition comprenant le ou les précurseurs de colorant avant mélange.

Ainsi, le procédé selon l'invention est mis en oeuvre en appliquant sur les fibres kératiniques, sèches ou humides, une composition obtenue par mélange extemporané, au moment de l'emploi, des trois compositions.

De préférence les rapports pondéraux R1 des quantités de compositions (A)+(B) / (C) et R2 des compositions (A) / (B) varient de 0,1 à 10 et de préférence de 0,3 à 5.

Selon un mode de réalisation préféré le rapport pondéral des quantités de compositions (C) / (A)+(B)+(C) varie de 0,4 à 0,7.

Le mélange présent sur les fibres est laissé en place pour une durée, en général, de l'ordre de 1 minute à 1 heure, de préférence de 10 minutes à 30 minutes.

La température durant le procédé est classiquement comprise entre la température ambiante (entre 15 à 25°C) et 80°C, de préférence entre la température ambiante et 60°C.

A l'issue du traitement, les fibres kératiniques humaines sont éventuellement rincées à l'eau, lavées au shampooing, rincées à nouveau à l'eau puis séchées ou laissées à sécher.

Comme indiqué auparavant, l'invention a aussi pour objet la composition prête à l'emploi comprenant un ou plusieurs composés organiques du silicium tels que définis précédemment et un ou plusieurs précurseurs de colorant, un ou plusieurs agents oxydants et un ou plusieurs polymères cationiques et / ou un ou plusieurs tensioactifs non ioniques oxyalkylénés ou glycérolés. De préférence, cette composition contient un ou plusieurs agents alcalins autres que les composés organiques du silicium.

La composition de coloration prête à l'emploi peut se présenter sous des formes diverses, telles que sous forme de liquides, de crèmes, de gels, ou sous toute autre forme appropriée pour réaliser une teinture des cheveux.

Enfin, l'invention concerne un dispositif à plusieurs compartiments comprenant dans un premier, une composition (A) substantiellement anhydre ou aqueuse présentant une teneur en eau inférieure à 5% en poids ou aqueuse présentant une teneur en eau supérieure à 5% en poids, comprenant un ou plusieurs corps gras exempts de groupe acide carboxylique et un ou plusieurs tensioactifs, dans un deuxième, une composition substantiellement anhydre ou aqueuse (B) présentant une teneur en eau inférieure à 5% en poids ou aqueuse présentant une teneur en eau supérieure à 5% en poids, comprenant un ou plusieurs composés de formule (I) et dans un troisième, une composition aqueuse (C) comprenant un ou plusieurs agents oxydants.

Les exemples suivants servent à illustrer l'invention sans toutefois présenter un caractère limitatif.

### EXEMPLES

### Exemple 1 : Composition éclaircissante

On prépare les compositions suivantes :

**Composition anhydre A (pourcentages exprimés en g%) :**

| | |
|---|---|
| Propylène carbonate | 1 |
| Octyldodécanol | 11,5 |
| Glycol distéarate | 8 |
| Laureth-2 | 1 |
| Polysorbate 21 | 11 |
| Disteardimonium hectorite | 3 |
| Huile de paraffine liquide | qsp 100g |

**Composition anhydre B (pourcentages exprimés en g%) :**

| | |
|---|---|
| Alcool éthylique dénaturé | 38,2 |
| Hexylène glycol | 12,9 |
| Dipropylène glycol | 12,9 |
| (3-aminopropyl)triéthoxysilane (*) | 36 |

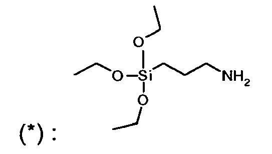

**Composition oxydante C (pourcentaqes exprimés en g%) :**

| | |
|---|---|
| Tocophérol | 0,1 |
| Stannate de sodium | 0,04 |
| Pentasodium pentetate | 0,06 |
| Polyquaternium-6 | 0,2 |
| Glycérine | 0,5 |
| Alcool Cétéarylique | 8 |
| Hexadimethrine Chloride | 0,15 |
| Ceteareth-33 | 3 |
| Acide phosphorique | Qs pH = 2.2 |
| Peroxyde d'hydrogène | 6 |
| Pyrophosphate tétrasodique | 0,03 |
| Huile de paraffine liquide | 20 |
| Amide d'acide de colza oxyéthyléné (40E) protégé | 1,20 |
| Eau déminéralisée | Qsp 100g |

Au moment de l'emploi, on mélange 10g de la composition (A) avec 4g de la composition (B) et 20g de la composition (C).

Le mélange obtenu (pH = 9,8 ± 0,1) est appliqué sur une mèche de cheveux défrisés d'une hauteur de ton de 4.

A noter que Le défrisage est réalisé avec le produit Dark and Lovely Super (de Softsheen Carson) pendant 20 minutes à 27°C sur plaque chauffante. Le rapport produit défrisant/mèche est de 10/1 (poids/poids) respectivement. A l'issue du traitement, les cheveux sont rincés à l'eau, lavés avec le shampooing Color Signal Neutralizing Shampoo (Dark and Lovely) puis rincés à l'eau et séchés.

Le temps de pause du mélange des compositions (A), (B) et (C) est de 30 minutes à 27°C sur plaque chauffante.

A la suite de ce temps de pause, la mèche est lavée avec du shampooing Elsève Multivitamines, puis est séchée au casque à 60°C.

La coloration des mèches est mesurée au moyen d'un colorimètre Konika Minolta CM2600D (observateur 10°, illuminant D65).

Comme le montre le tableau ci-dessous, on obtient un bon niveau d'éclaircissement. De plus, la mèche présente un toucher cosmétique doux et lisse.

Enfin, l'application est agréable car sans odeur agressive.

| | **L*** | **a*** | **b*** | Δ**E*ab** |
|---|---|---|---|---|
| **Cheveu châtain défrisé non traité** | 17.65 | 1.88 | 1.96 | |
| **Cheveu traité avec le mélange l'invention** | 18.32 | 4.84 | 5.21 | 4.44 |

### Exemple 2 : Composition colorante

On prépare les compositions suivantes :

**Composition anhydre (A) (pourcentages exprimés en g%) :**

| | |
|---|---|
| Propylène carbonate | 1 |
| Octyldodécanol | 11,5 |
| Glycol distéarate | 8 |
| Laureth-2 | 1 |
| Polysorbate 21 | 11 |
| Disteardimonium hectorite | 3 |
| Huile de paraffine liquide | qsp 100g |

**Composition anhydre (B) (pourcentages exprimés en g%) :**

| | |
|---|---|
| Alcool éthylique dénaturé pur | 8,8 |
| Hexylène glycol | 3 |
| Dipropylène glycol | 3 |
| (3-aminopropyl)triéthoxysilane | 36 |
| Ceteareth-33 | 21,25 |
| Para-phénylènediamine | 0,33 |
| 2-méthyl-5-hydroxyéthylaminophénol | 4,08 |
| 5-amino-6-chloro-o-crésol | 2,72 |
| Para-aminophénol | 3,4 |
| Palmitate d'ascorbyle | 0,2 |
| Condensat d'oxyde d'éthylène et d'oxyde de propylène et d'oxyde d'éthylène. PM :2900 g/mol ; 13OE/30OP/13OE | qsp 100 |

**Composition oxydante (C) (pourcentages exprimés en g%) :**

| | |
|---|---|
| Tocophérol | 0,1 |
| Sodium stannate | 0,04 |
| Pentasodium pentétate | 0,06 |
| Polyquaternium-6 | 0,2 |
| Glycérine | 0,5 |
| Alcool cétéarylique | 8 |
| Hexadimethrine Chloride | 0,15 |
| Ceteareth-33 | 3 |
| Acide phosphorique | 0 |
| Peroxyde d'hydrogène | 6 |
| Pyrophosphate tétrasodique | 0,03 |
| Huile de paraffine liquide | 20 |
| Amide d'acide de colza oxyéthyléné (4OE) protégé | 1,20 |
| Eau déminéralisée | Qsp 100g |

Au moment de l'emploi, on mélange 10 g de la composition (A) avec 4 g de la composition (B) et 20 g de la composition (C).

Le mélange obtenu (pH = 9,8 ± 0,1) est appliqué sur une mèche de cheveux naturels à 90 % de blanc.

Le temps de pause du mélange des compositions (A), (B) et (C) est de 30 minutes à 27°C sur plaque chauffante.

A la suite de ce temps de pause, la mèche est lavée avec du shampooing Elsève Multivitamines, puis est séchée au casque à 60°C.

La coloration des mèches est mesurée au moyen d'un colorimètre Konika Minolta CM2600D (observateur 10°, illuminant D65).

Comme le montre le tableau ci-dessous, on obtient un cuivré rouge avec une bonne couverture des cheveux blancs.

| | **L*** | **a*** | **b*** | Δ**E*ab** |
|---|---|---|---|---|
| Cheveu naturel à 90% blancs (BN) BN | 60.41 | 0.35 | 15.41 | - |
| traité avec le mélange l'invention | 16.71 | 30.85 | 28.54 | 54.89 |

### Exemple 3 : composition éclaircissante

Les compositions suivantes sont préparées :

**Composition anhydre A1 (pourcentages exprimés en g%) :**

| | |
|---|---|
| Propylène carbonate | 1 |
| Octyldodécanol | 11,5 |
| Glycol distéarate | 8 |
| Laureth-2 | 1 |
| Polysorbate 21 | 11 |
| Disteardimonium hectorite | 3 |
| Huile de paraffine liquide | qsp 100g |

**Composition aqueuse B1 (pourcentages exprimés en g%) :**

| | |
|---|---|
| Alcool éthylique | 8,8 |
| Hexylène glycol | 3 |
| Propylène glycol | 6,2 |
| Dipropylène glycol | 3 |
| Acide diethylène triamine pentacétique à 40% dans l'eau | 1 |
| Acide ascorbique | 0.25 |
| Métabisulfite de sodium | 0.7 |
| (3-aminopropyl)triéthoxysilane (*) | 10,87 |
| Monoéthanolamine pure | 15.05 |
| Eau déminéralisée | qsp 100 |

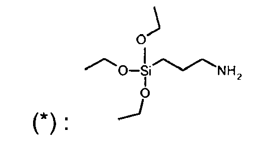

**Composition oxydante (C) (pourcentages exprimés en g%) :**

| *Emulsion A (préparée par le procédé PIT)* | | |
|---|---|---|
| Phase I | Sorbitol cristallisé | 5 |
| | Huile de paraffine liquide | 60,2 |
| | Eau déminéralisée | 16,625 |
| | Alcool béhénique oxyéthyléné 100E | 6 |
| | Acide phorsphorique | 0,15 |
| Phase II | Acide etidronique, sel tetrasodique en solution aqueuse à 30% | 0,2 |
| | Pyrophosphate tetra-sodique, 10H2O | 0,04 |
| | Salicylate de sodium | 0,035 |
| | Polycondensat tetramethyl hexamethylenediamine / dichloro 1,3-propylène en solution aqueuse | 0,25 |
| | Chlorure de polydimethyl di-allyl ammonium dans l'eau à 40% non stabilisé | 0,5 |
| | Alcool éthylique 96° dénaturé | 2 |
| | Condensat d'oxyde d'éthylène et d'oxyde de propylène et d'oxyde d'éthylène. PM :2900 g/mol ; 13OE/30OP/13OE | 3 |
| Phase III | Peroxyde d'hydrogène | 6 |

### Procédé de fabrication de l'émulsion

- On chauffe les ingrédients de la phase I au bain marie sous agitation de type Rayneri (400 tr/min). On obtient une émlusion blanche fluide qui devient translucide vers 68°C et s'épaissit au-delà.
- Dès que l'émulsion s'épaissit, on retire le bain marie et on laisse refroidir sous même agitation
- A 40°C, on introduit les ingrédients de la phase II
- Enfin, on introduit le peroxyde d'hydrogène
- La perte de poids en eau est compensée (< 5%)
L'émulsion obtenue est une crème épaisse, de pH = 3

### Application de la composition

Au moment de l'emploi, on mélange 10 g de la composition (A1) avec 4 g de la composition (B1) et 15 g de la composition (C1).

Le mélange obtenu (pH = 10 ± 0,1) est appliqué sur une mèche de cheveux défrisés d'une hauteur de ton de 4.

A noter que Le défrisage est réalisé avec le produit Dark and Lovely Super (de Softsheen Carson) pendant 20 minutes à 27°C sur plaque chauffante. Le rapport produit défrisant/mèche est de 10/1 (poids/poids) respectivement. A l'issue du traitement, les cheveux sont rincés à l'eau, lavés avec le shampooing Color Signal Neuralizing Shampoo (Dark and Lovely) puis rincés à l'eau et séchés.

Le temps de pause du mélange des compositions (A1), (B1) et (C1) sur les cheveux défrisés est de 30 minutes à 27°C sur plaque chauffante.

A la suite de ce temps de pause, la mèche est lavée avec du shampooing Elsève Multivitamines, puis est séchée au casque à 60°C.

### Résultats

La coloration des mèches est mesurée au moyen d'un colorimètre Konika Minolta CM2600D (observateur 10°, illuminant D65).

Comme le montre le tableau ci-dessous, on obtient un bon niveau d'éclaircissement. De plus, la mèche présente un toucher cosmétique, doux et lisse.

Enfin, l'application est agréable car sans odeur agressive.

| | **L*** | **a*** | **b*** | Δ**E*ab** |
|---|---|---|---|---|
| Cheveux châtains défrisés | **17,54** | **1,92** | **1,83** | / |
| Cheveux traités avec le mélange l'invention | **20,25** | **5,48** | **6,07** | **6.17** |

### Exemple 4 : Composition colorante

On prépare les compositions suivantes :

**Composition anhydre (A2) (pourcentages exprimés en g%) :**

| | |
|---|---|
| Propylène carbonate | 1 |
| Octyldodécanol | 11,5 |
| Glycol distéarate | 8 |
| Laureth-2 | 1 |
| Polysorbate 21 | 11 |
| Disteardimonium hectorite | 3 |
| Huile de paraffine liquide | qsp 100g |

**Composition anhydre (B2) (pourcentages exprimés en g%) :**

| | |
|---|---|
| Alcool éthylique dénaturé | 8,8 |
| Hexylène glycol | 3 |
| Propylène glycol | 6.2 |
| Dipropylène glycol | 3 |
| Acide diethylène triamine pentacétique à 40% dans l'eau | 1 |
| Acide ascorbique | 0,25 |
| Métabisulfite de sodium | 0,7 |
| (3-aminopropyl)triéthoxysilane | 10,87 |
| Monoéthanolamine pure | 15,05 |
| Para-phénylène diamine | 0,28 |
| Para-aminophénol | 3,48 |
| 1-méthyl-2-hydroxy-4-β-hydroxyéthylamino-benzène | 2,9 |
| 5- amino-6-chloro-o-cresol purifié | 2,32 |
| Eau déminéralisée | qsp 100g |

**Composition oxydante (C2) (pourcentages exprimés en g%) :**

| | |
|---|---|
| Tocophérol | 0,1 |
| Sodium stannate | 0,04 |
| Pentasodium pentétate | 0,06 |
| Polyquaternium-6 | 0,2 |
| Glycérine | 0,5 |
| Alcool cétéarylique | 8 |
| Hexadimethrine Chloride | 0,15 |
| Ceteareth-33 | 3 |
| Acide phosphorique | 0 |
| Peroxyde d'hydrogène | 6 |
| Pyrophosphate tétrasodique | 0,03 |
| Huile de paraffine liquide | 20 |
| Amide d'acide de colza oxyéthyléné (4OE) protégé | 1,20 |
| Eau déminéralisée | Qsp 100g |

La composition C2 est réalisée par mélange à chaud des composés non hydrosolubles (phase grasse) puis addition de la phase aqueuse constituée de l'eau et des composés hydrosolubles.

### Application de la composition

Au moment de l'emploi, on mélange 10 g de la composition (A2) avec 4 g de la composition (B2) et 15 g de la composition (C2).

Le mélange obtenu (pH = 10,1 ± 0,1) est appliqué sur une mèche de cheveux naturels à 90 % de blancs.

Le temps de pause du mélange des compositions (A2), (B2) et (C2) est de 30 minutes à 27°C sur plaque chauffante.

A la suite de ce temps de pause, la mèche est lavée avec du shampooing Elsève Multivitamines, puis est séchée au casque à 60°C.

### Résultats

La coloration des mèches est mesurée au moyen d'un colorimètre Konika Minolta CM2600D (observateur 10°, illuminant D65).

Comme le montre le tableau ci-dessous, on obtient un cuivré rouge avec une bonne couverture des cheveux blancs.

| | **L*** | **a*** | **b*** | Δ**E*ab** |
|---|---|---|---|---|
| **Cheveu naturel à 90% blancs (BN)** | 52,42 | 1,53 | 12,41 | / |
| **BN traité avec le mélange l'invention** | 25,85 | 16,24 | 12,16 | 30,37 |

### Exemple 5 : composition colorante

Les compositions suivantes sont réalisées (quantité en gramme de matière active) :

**Composition aqueuse contenant au moins un composé organique du silicium :**

| | |
|---|---|
| Acide lactique | 10,8 |
| Hydroxyéthylcellulose (Natrosol 250 HHR d'AQUALON) | 0,4 |
| 3-Aminopropyltriéthoxysilane Dow Corning Z-6011 Silane | 30 |
| Eau | Qsp 100 |

**Composition contenant des colorants d'oxydation et un agent alcalin :**

| | |
|---|---|
| Acide oleique | 2,7 |
| Hydroxyde d'ammonium Hydroxyde d'ammonium | 2,22 (exprimée en NH₃) |
| Pentasodium pentetate | 0,8 |
| Monoétanolamine | 0,63 |
| 2-Oleamido-1,3-Octadecanediol | 0,01 |
| 2,5-Diaminotoluène | 0,7623 |
| Resorcinol | 0,66 |
| m-Aminophenol | 0,14 |
| 2,4-Diaminophenoxyethanol 2HCl | 0,02 |
| Alcool cetearylique | 16,2 |
| Alcool oleique | 2,7 |
| Chlorure d'Hexadimethrine(Mexomere PO de CHIMEX) | 3 |
| Oleth-30 | 3,6 |
| Metabisulfitede sodium | 0,71 |
| Parfum | 0,5 |
| Eau | Qsp 100 |

**Composition contenant de l'eau oxygénée :**

| | |
|---|---|
| Trideceth Carboxamide Mea | 0,85 |
| Stannate de sodium | 0,04 |
| Pentasodium pentetate | 0,06 |
| Glycerine | 0,5 |
| Alcool cétéarylique | 2,28 |
| Ceteareth-25 | 0,57 |
| Peroxyde d'hydrogène | 6 |
| Pyrophosphate tetrasodique | 0,02 |
| Acide phosphorique | Qs pH2 |
| Eau | Qsp 100 |

La composition contenant des colorants d'oxydation et un agent alcalin est diluée extemporanément avec 1 fois et demi son poids de la composition comprenant l'agent oxydant.

La composition aqueuse contenant au moins un composé organique du silicium est introduite dans le mélange précédent à hauteur de 6 grammes pour 120 grammes de mélange précédent.

Ce mélange est ensuite appliqué sur des cheveux chatains fins.

Après un temps de pause de 30 minutes à température ambiante , les cheveux sont rincés, lavés avec un shampooing standard et séchés.

Après décoloration et coloration des cheveux, on obtient au final des cheveux teints dans une nuance chatain clair. La chevelure présente des propriétés coiffantes marquées avec beaucoup de volume et est plus corporisée.

## Revendications

1. Procédé de coloration et/ou d'éclaircissement des fibres kératiniques humaines, dans lequel on met en contact lesdites fibres avec :
*une composition (A) substantiellement anhydre présentant une teneur en eau inférieure à 5% en poids ou aqueuse présentant une teneur en eau supérieure à 5% en poids, comprenant un ou plusieurs corps gras exempts de groupe acide carboxylique, un ou plusieurs tensioactifs,
* une composition (B) substantiellement anhydre présentant une teneur en eau inférieure à 5% en poids ou aqueuse présentant une teneur en eau supérieure à 5% en poids, comprenant un ou plusieurs composés aminosiliciés ne comprenant qu'un atome de silicium portant trois groupements alcoxy en C₁-C₄, de formule suivante (I) : 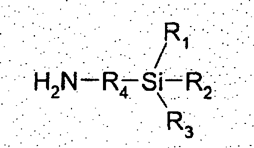 formule dans laquelle
- R₁, R₂, R₃ sont identiques R₄ est un radical divalent de structure : 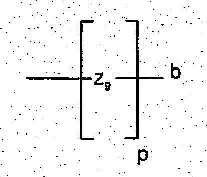 Dans laquelle :
■ Z₉ désigne un radical alkylène linéaire en C₁-C₂₀
■
■ p vaut 1
■ b représente la liaison à l'atome d'azote du groupement amino,
* une composition aqueuse (C) comprenant un ou plusieurs agents oxydants.

2. Procédé selon la revendication 1, caracténsé en ce que la composition (A) et / ou (B) comprend un ou plusieurs précurseurs de colorant d'oxydation choisis parmi les bases et les coupleurs d'oxydation ; un ou plusieurs colorants directs, ou leurs mélanges

3. Procédé selon la revendication 1 ou 2, **caractérisé en ce que** le corps gras est un composé choisi parmi un alcool gras, un ester d'acide gras, un ester d'alcool gras, une huile minérale, végétale, animale ou synthétique, une silicone ou une cire.

4. Procédé selon l'une des revendications précédentes, caracténsé en ce que le corps gras est choisi parmi l'huile de vaseline, l'huile de paraffine, les polydécènes, les esters d'acides gras, ou leurs mélanges.

5. Procédé selon l'une quelconque des revendications précédentes, **caractérisé en ce que** la composition (A) et / ou (B) comprend un agent alcalin différent des composés de formule (I).

6. Procédé selon la revendication 5, caracténsé en ce que l'agent alcalin est choisi parmi les amines organiques dont le pKb à 25°C est inférieur à 12 et leurs sels ; les bases inorganiques possédant dans leur structure un ou plusieurs éléments des colonnes 1 à 13 du tableau périodique des éléments autre que l'hydrogène, ne comportant pas simultanément d'atome(s) de carbone et d'hydrogène ; les sels d'ammonium choisis parmi les sels d'acide suivants : acétate, carbonate, bicarbonate, chlorure, citrate, nitrate, nitrite, phosphate, sulfate

7. Procédé selon l'une des revendications 5 ou 6, caracténsé en ce que l'agent alcalin est choisi parmi les alcanolamines, les acides aminés basiques et les hydroxydes ou carbonates de métaux alcalins, ou leurs mélanges.

8. Procédé selon l'une quelconque des revendications précédentes, **caractérisé en ce que** l'on applique sur les fibres kératiniques, une composition obtenue par mélange extemporané, au moment de l'emploi, des compositions (A), (B) et (C).

9. Composition prête à l'emploi comprenant un ou plusieurs précurseurs de colorants tels que définis dans la revendication 2, un ou plusieurs composés organiques du silicium tels que définis dans la revendication 1, un ou plusieurs agents oxydants et un ou plusieurs polymères cationiques et / ou un ou plusieurs tensioactifs non ioniques oxyalkylénés ou glycérolés.

10. Composition selon la revendication 9 comprenant un ou plusieurs agents alcalins autres que les composés organiques du silicium.

11. Dispositif à plusieurs compartiments comprenant dans un premier, une composition (A) substantiellement anhydre présentant une teneur en eau inférieure à 5% en poids ou aqueuse présentant une teneur en eau supérieure à 5% en poids, comprenant un ou plusieurs corps gras exempts de groupe acide carboxylique et un ou plusieurs tensioactif, dans un deuxième, une composition (B) substantiellement anhydre présentant une teneur en eau inférieure à 5% en poids ou aqueuse présentant une teneur en eau supérieure à 5% en poids, comprenant un ou plusieurs composés aminosiliciés ne comprenant qu'un atome de silicium portant trois groupements alcoxy en C₁-C₄, de formule (I) et dans un troisième, une composition aqueuse (C) comprenant un ou plusieurs agents oxydants ; la formule (I) étant la suivante : 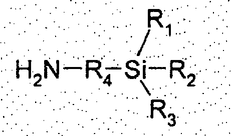 formule dans laquelle :
R_{1,} R₂, R₃ sont identiques
R₄ est un radical divalent de structure : 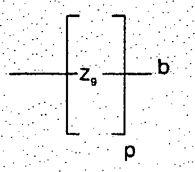
Dans laquelle :
■ Z₉ désigne un radical alkylène linéaire en C₁-C₂₀
■ p vaut 1
■ b représente la liaison à l'atome d'azote du groupement amino

## Patentansprüche

1. Verfahren zum Färben und/oder Aufhellen von menschlichen Keratinfasern, bei dem man die Fasern mit
* einer Zusammensetzung (A), die weitgehend wasserfrei ist und einen Wassergehalt von weniger als 5 Gew.-% aufweist oder wässrig ist und einen Wassergehalt von mehr als 5 Gew.-% aufweist und eine oder mehrere Fettsubstanzen, die keine Carbonsäuregruppe aufweisen, und ein oder mehrere Tenside umfasst;
* einer Zusammensetzung (B), die weitgehend wasserfrei ist und einen Wassergehalt von weniger als 5 Gew.-% aufweist oder wässrig ist und einen Wassergehalt von mehr als 5 Gew.-% aufweist und eine oder mehrere Aminosiliciumverbindungen, die nur ein Siliciumatom mit drei C₁-C₄-Alkoxygruppen umfassen und die folgende Formel (I) aufweisen: 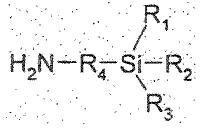 worin:
- R₁, R₂ und R₃ gleich sind und R₄ für einen zweiwertigen Rest der Struktur: 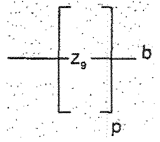 worin:
■ Z₉ für einen linearen C₁-C₂₀-Alkylenrest steht,
■ p gleich 1 ist,
■ b für die Bindung an das Stickstoffatom der Aminogruppe steht,
steht, umfasst,
* einer wässrigen Zusammensetzung (C), die ein oder mehrere Oxidationsmittel umfasst,
in Berührung bringt.

2. Verfahren nach Anspruch 1, **dadurch gekennzeichnet, dass** die Zusammensetzung (A) und/oder (B) eine oder mehrere Oxidationsfarbstoffvorstufen, die aus Oxidationsbasen und Oxidationskupplern ausgewählt sind, einen oder mehrere Direktfarbstoffe oder Mischungen davon umfasst.

3. Verfahren nach Anspruch 1 oder 2, **dadurch gekennzeichnet, dass** es sich bei der Fettsubstanz um eine Verbindung handelt, die aus einem Fettalkohol, einem Fettsäureester, einem Fettalkoholester, einem Mineralöl, einem pfanzlichen Öl, einem tierischen Öl, einem synthetischen Öl, einem Silikon oder einem Wachs ausgewählt ist.

4. Verfahren nach einem der vorhergehenden Ansprüche, **dadurch gekennzeichnet, dass** die Fettsubstanz aus Vaselineöl, Paraffinöl, Polydecenen, Fettsäureestern oder Mischungen davon ausgewählt ist.

5. Verfahren nach einem der vorhergehenden Ansprüche, **dadurch gekennzeichnet, dass** die Zusammensetzung (A) und/oder (B) ein alkalisches Mittel, das sich von den Verbindungen der Formel (I) unterscheidet, umfasst.

6. Verfahren nach Anspruch 5, **dadurch gekennzeichnet, dass** das alkalische Mittel aus organischen Aminen mit einem pK_{b}-Wert bei 25°C von weniger als 12 und Salzen davon; anorganischen Basen, die in ihrer Struktur ein oder mehrere Elemente aus den Spalten 1 bis 13 des Periodensystems der Elemente außer Wasserstoff aufweisen und nicht gleichzeitig Kohlenstoff- und Wasserstoffatome umfassen; und Ammoniumsalzen, die aus den folgenden Säuresalzen ausgewählt sind: Acetat, Carbonat, Hydrogen-carbonat, Chlorid, Citrat, Nitrat, Nitrit, Phosphat, Sulfat, ausgewählt ist.

7. Verfahren nach einem der Ansprüche 5 oder 6, **dadurch gekennzeichnet, dass** das alkalische Mittel aus Alkanolaminen, basischen Aminosäuren und Alkalimetallhydroxiden oder -carbonaten oder Mischungen davon ausgewählt ist.

8. Verfahren nach einem der vorhergehenden Ansprüche, **dadurch gekennzeichnet, dass** man auf die Keratinfasern eine durch unmittelbares Mischen der Zusammensetzungen (A), (B) und (C) zum Zeitpunkt der Anwendung erhaltene Zusammensetzung aufbringt.

9. Gebrauchsfertige Zusammensetzung, umfassend eine oder mehrere Farbstoffvorstufen gemäß Anspruch 2, eine oder mehrere Organosiliciumverbindungen gemäß Anspruch 1, ein oder mehrere Oxidationsmittel und ein oder mehrere kationische Polymere und/oder ein oder mehrere oxyalkylenierte oder glycerinierte nichtionisches Tenside.

10. Zusammensetzung nach Anspruch 9, die ein oder mehrere alkalische Mittel, die von den Organo-siliciumverbindungen verschieden sind, umfasst.

11. Vorrichtung mit mehreren Kompartimenten, umfassend in einem ersten Kompartiment eine Zusammensetzung (A), die weitgehend wasserfrei ist und einen Wassergehalt von weniger als 5 Gew.-% aufweist oder wässrig ist und einen Wassergehalt von mehr als 5 Gew.-% aufweist und eine oder mehrere Fettsubstanzen, die keine Carbonsäuregruppe aufweisen, und ein oder mehrere Tenside umfasst, in einem zweiten Kompartiment eine Zusammensetzung (B), die weitgehend wasserfrei ist und einen Wassergehalt von weniger als 5 Gew.-% aufweist oder wässrig ist und einen Wassergehalt von mehr als 5 Gew.-% aufweist und eine oder mehrere Amino-siliciumverbindungen, die nur ein Siliciumatom mit drei C₁-C₄-Alkoxygruppen umfassen und die Formel (I) aufweisen, umfasst, und in einem dritten Kompartiment eine wässrige Zusammensetzung (C), die ein oder mehrere Oxidationsmittel umfasst; wobei die Formel (I) die folgende ist: 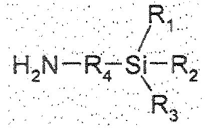 worin:
R₁, R₂ und R₃ gleich sind und
R₄ für einen zweiwertigen Rest der Struktur: 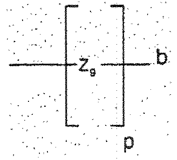 worin:
■ Z₉ für einen linearen C₁-C₂₀-Alkylenrest steht,
■ p gleich 1 ist,
■ b für die Bindung an das Stickstoffatom der Aminogruppe steht,
steht.

## Claims

1. Process for colouring and/or bleaching human keratin fibres, in which said fibres are put in contact with:
*a substantially anhydrous composition (A) having a water content less than 5% by weight or aqueous composition having a water content greater than 5% by weight, comprising one or more fatty substances free from carboxylic acid groups, one or more surfactants,
* a substantially anhydrous composition (B) having a water content less than 5% by weight or aqueous composition having a water content greater than 5% by weight, comprising one or more amino silicone compounds only comprising one silicon atom bearing three C₁-C₄ alkoxy groups, having the following formula (I) : 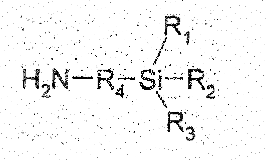 formula in which:
- R₁, R₂, R₃ are identical
- R₄ is a divalent substituent having the structure: 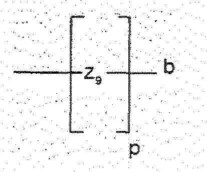
In which:
• Z₉ denotes a C₁-C₂₀ linear alkylene substituent
• p equals 1
• b represents the bond to the nitrogen atom of the amino group,
* an aqueous composition (C) comprising one or more oxidizing agents.

2. Process according to Claim 1, **characterized in that** the composition (A) and/or (B) comprises one or more oxidative dye precursors chosen from oxidation bases and couplers; one or more direct dyes, or mixtures thereof.

3. Process according to Claim 1 or 2, **characterized in that** the fatty substance is a compound chosen from a fatty alcohol, a fatty acid ester, a fatty alcohol ester, a mineral, plant, animal or synthetic oil, a silicone compound or a wax.

4. Process according to any one of the previous claims, **characterized in that** the fatty substance is chosen among liquid petrolatum, liquid paraffin, polydecenes, fatty acid esters, or mixtures thereof.

5. Process according to any one of the previous claims, **characterized in that** the composition (A) and/or (B) comprises an alkaline agent different than the compounds having formula (I).

6. Process according to Claim 5, **characterized in that** the alkaline agent is chosen from organic amines whose pKb at 25°C is less than 12 and the salts thereof; inorganic bases having in their structure one or more elements from columns 1 to 13 of the periodic table of the elements other than hydrogen, not simultaneously containing atom(s) of carbon and hydrogen; ammonium salts chosen from the following acid salts: acetate, carbonate, bicarbonate, chloride, citrate, nitrate, nitrite, phosphate, sulfate.

7. Process according to one of Claims 5 and 6, **characterized in that** the alkaline agent is chosen from alkanolamines, basic amino acids and alkali metal hydroxides or carbonates, or mixtures thereof.

8. Process according to any one of the previous claims, **characterized in that** a composition obtained by extemporaneous mixing at the time of use of compositions (A), (B) and (C) is applied to the keratin fibres.

9. Ready-to-use composition comprising one or more dye precursors as defined in Claim 2, one or more organosilicon compounds as defined in Claim 1, one or more oxidizing agents and one or more cationic polymers and/or one or more oxyalkylenated or glycerolated non-ionic surfactants.

10. Composition according to Claim 9 comprising one or more alkaline agents other than organosilicon compounds.

11. Device with several compartments comprising in a first, a substantially anhydrous composition (A) having a water content less than 5% by weight or aqueous composition having a water content greater than 5% by weight, comprising one or more fatty substances free from carboxylic acid groups and one or more surfactants, in a second, a substantially anhydrous composition (B) having a water content less than 5% by weight or aqueous composition having a water content greater than 5% by weight, comprising one or more amino silicone compounds only comprising one silicon atom bearing three C₁-C₄ alkoxy groups, having the formula (I) and in a third, an aqueous composition (C) comprising one or more oxidizing agents; formula (I) being as follows: 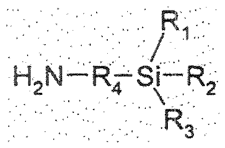 formula in which:
R₁, R₂, R₃ are identical
R₄ is a divalent substituent having the structure: 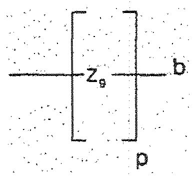 In which:
• Z₉ denotes a C₁-C₂₀ linear alkylene substituent
• p equals 1
• b represents the bond to the nitrogen atom of the amino group.
